# EUROPEAN PATENT APPLICATION

(11) **EP 1 500 709 A1**
(43) Date of publication of application: **26.01.2005**
(21) Application number: 03077299.0
(22) Date of filing: 21.07.2003
(51) Int. Cl.: C12Q 1/68

(54) **Means and methods for detecting a risk of infarction related to atherosclerosis**

(71) Applicant: Academisch Ziekenhuis bij de Universiteit van Amsterdam, 1105 AZ Amsterdam ZO (NL); Leids Universitair Medisch Centrum, 2333 ZA Leiden (NL)
(72) Inventor: Reitsma, Pieter Hendrik, 2333 XJ Leiden (NL); Rosendaal, Frits Richard, 2241 KC Wassenaar (NL)
(74) Representative: Prins, Adrianus Willem, Mr. Ir.

(57) **Abstract**

The invention provides a method for determining whether a subject is at risk of undergoing an infarction related to atherosclerosis comprising measuring an inflammation parameter in a sample obtained from said subject and determining whether the value of said inflammation parameter is indicative for said risk. Said sample preferably comprises a whole blood sample. More preferably, said inflammation parameter comprises an inflammation parameter of a cell and/or derivative thereof present in said sample, such as the amount of protein and/or RNA present upon and/or within such cells/derivatives. Additionally, an excreted product produced by said cell/derivative may be measured. The invention furthermore provides a method for quantifying at least one target nucleic acid in a sample, comprising incubating said sample with at least one probe capable of annealing to said target nucleic acid, amplifying annealed probes and quantifying said probes, characterized in that said sample comprises a whole blood sample.

## Description

The invention relates to the field of medicine. More specifically the invention relates to diagnosis and/or prediction of disease, such as atherosclerosis.

Atherosclerosis is the leading cause of illness and death in most Western countries. It involves deposits of fatty substances, cholesterol, cellular waste products, calcium, and/or fibrin in the inner lining of an artery. Atherosclerosis can affect the brain, heart, kidneys, other vital organs, and/or extremities. It may lead to strokes, coronary heart diseases such as myocardial infarction or coronary artery disease, etcetera. Atherosclerosis is a specific type of arteriosclerosis, meaning loss of vessel elasticity.

Atherosclerosis is a complex process. It is not known exactly how it begins or what causes it. Some theories have been proposed in the art. Many scientists think atherosclerosis begins when the innermost layer of the artery, the endothelium, becomes damaged. Over time, fats, cholesterol, fibrin, platelets, cellular debris and calcium are deposited in the artery wall. These substances may stimulate the cells of the artery wall to produce still other substances that result in further accumulation of cells in the innermost layer of the artery wall where atherosclerotic lesions form. These cells accumulate and many of them divide. At the same time, fat builds up within these cells and around them. They also form connective tissue. The innermost layer of the artery becomes markedly thickened by these accumulating cells and surrounding material. If the wall is thickened sufficiently, the diameter of the artery will be reduced and the amount of blood decreased, thus decreasing the oxygen supply. Usually, atherosclerosis does not produce symptoms until it severely narrows an artery, and/or until it causes a sudden obstruction. As atherosclerosis severely narrows at least one artery, the areas of the body it serves may not receive enough blood, which carries oxygen to the tissues. The first symptom of a narrowing artery may be pain or cramps at times when the blood flow cannot keep up with the body's demand for oxygen. For instance, during exercise, a person may feel chest pain (angina) because of a lack of oxygen to the heart, or while walking, a person may feel leg cramps (intermittent claudication) because of a lack of oxygen to the legs. Typically, these symptoms develop gradually as an atheroma slowly narrows an artery. However, when an obstruction occurs suddenly, for instance when a blood clot lodges in an artery, symptoms come on suddenly. If the oxygen supply to the heart muscle is reduced, a myocardial infarction can occur. If the oxygen supply to the brain is cut off, a stroke can occur. And if the oxygen supply to the extremities is reduced, gangrene can result.

Inflammation plays a key role in the initiation and progression of atherosclerosis and in the development of acute coronary events [Libby P. Inflammation in atherosclerosis. Nature. 2002;420:868-74]. Activated leucocytes, cytokines and chemokines are prominent features of an atherosclerotic plaque. Moreover, plasma protein levels of several markers of inflammation such as cell adhesion molecules, cytokines, pro-atherogenic enzymes and C-reactive protein (CRP) were found to be involved with cardiovascular events in a variety of clinical settings [Blake GJ, Ridker PM. Inflammatory bio-markers and cardiovascular risk prediction. J Intern Med. 2002;252:283-94).

Whereas the importance of local inflammation in the vessel wall in individuals with atherosclerosis has been established, the presence of a chronic systemic inflammatory state remains unclear. An inflammatory state may be the by-product of the local inflammation in the vessel wall, or it may reflect an active or latent infection that is in part responsible for the atherosclerotic process.

In spite of various research in the art, it is often not possible to accurately predict whether an individual will develop a disease such as atherosclerosis.

It is an aim of the present invention to provide a method for determining whether a subject is at risk of undergoing an infarction related to atherosclerosis. It is a further object of the invention to provide new methods for identifying at least one parameter of a disease.

The invention provides a method for determining whether a subject is at risk of undergoing an infarction related to atherosclerosis comprising measuring an inflammation parameter in a sample obtained from said subject and determining whether the value of said inflammation parameter is indicative for said risk. Said sample preferably comprises a whole blood sample, since said sample is easy to obtain and can be directly used in a method of the invention.

More preferably, a method of the invention is provided wherein said inflammation parameter comprises an inflammation parameter of a cell and/or derivative thereof present in said sample. The present inventors have found that inflammation parameters of circulating cells are particularly indicative for a risk of an infarction related to atherosclerosis. Preferably, said cells comprise leukocytes. Plasma protein levels, which are currently measured, do not fully reflect the inflammatory signature of circulating cells such as leukocytes in whole blood because tissue leucocytes may also contribute to the plasma protein composition, and many inflammatory mediators are also produced by other cell-types. Plasma protein levels are therefore often not fully related to the inflammatory signature of circulating cells.

By an infarction related to atherosclerosis is meant herein a disorder which at least in part results from a thickened artery. An infarction related to atherosclerosis often involves a part of an individual's body which is at least in part deprived of oxygen. During a process of thickening of an artery, dependent tissue may already be deprived of oxygen to some extent. If an attack occurs, at least part of an artery is essentially completely blocked. Tissue that normally acquires its oxygen from such artery will typically die, unless oxygen supply is compensated from another source. Examples of infarctions related to atherosclerosis are myocardial infarction and stroke. However, a method of the invention is also suitable for diagnosis of non-acute phases of atherosclerosis. A process of reduction of artery diameter can be diagnosed as well.

A subject is defined herein as any animal comprising blood vessels. A subject may also be referred to with the term "individual". Preferably, said animal comprises a mammal. In a preferred embodiment, said subject comprises a human. Preferably, said subject is devoid of other inflammation events such as infection and/or autoimmune disease, because such other inflammation events could hamper diagnosis of a risk of infarction related to atherosclerosis. Subclinical manifestations of other infections can typically be accounted for by sampling at different time points. If another inflammation event does occur, it is preferred to perform a method of the invention at another time point and/or with a combination of at least two inflammation parameters, as will be discussed below.

With an inflammation parameter of a cell and/or derivative thereof is meant a parameter that is derived from, and/or measured upon, said cell and/or derivative. Such parameter preferably comprises the amount of protein and/or RNA present upon and/or within such cells/derivatives. Additionally, an excreted product produced by said cell/derivative may be measured.

By a derivative of a cell is meant herein a part of a cell originating from a cell. Said cell may for instance have been damaged, after which cell contents may be distributed throughout said sample. Said cell may have been damaged in a subject before said sample was obtained. Said cell may however also have been damaged during, or after, provision of said sample. Said cell may for instance have been damaged because of harsh (mechanical) treatment of said sample, because of an action of cellulases, etcetera. A derivative of a cell for instance comprises a nucleus, (part of) an organelle, and/or a cell membrane.

Said cell preferably comprises a leukocyte. Leukocytes comprise granulocytes, lymphocytes and/or monocytes or macrophages. More preferably, said cell comprises a granulocyte and/or monocyte or macrophage. According to the invention, an expression profile of at least one inflammatory parameter of a leukocyte is indicative for the presence of, or risk of, atherosclerosis and an infarction related to atherosclerosis. An expression profile of leukocytes is sufficient for a diagnosis according to the invention. This allows a use of a whole blood sample for determining whether a value of an inflammation parameter is indicative for disease. A whole blood sample is preferred because it is easy to obtain, it involves little discomfort to a subject and it can be directly used in a method of the invention.

An inflammation parameter may be any expression product, or functional part thereof, from a gene involved in inflammation. For instance, said inflammation parameter may be the presence, or relative amount, of a protein or RNA. Preferably, said inflammation parameter involves a combination of several proteins and/or several kinds of RNA, most preferably a combination of two, three or four proteins and/or RNA sequences.

A value of an inflammation parameter is defined as an outcome of a measurement of said parameter. Depending on the kind of parameter that is measured, said value can be of different kinds. Said value may for instance be an amount of protein and/or RNA. Upregulation, or downregulation, of said protein and/or RNA can be indicative for an infarction related to atherosclerosis. Mostly, said amount is measured as a relative amount, for instance by comparing the amount of said protein/RNA with the amount of another protein/RNA sequence. Preferably, said other protein/RNA sequence is an abundant protein/RNA sequence. Said relative amount can also be determined by comparing the amount of said protein/RNA with the total amount of protein/RNA in said sample. Several ways of measuring (relative) amounts of proteins/nucleic acids are known in the art. In one embodiment, (amplified) target nucleic acids are provided with a fluorescent label. An amount of said target nucleic acid is subsequently determined by measuring the corresponding fluorescence intensity. Such intensity is often compared with an internal standard, and/or the intensity of a second fluorescence label. Of course, many alternative methods for estimating a (relative) amount of a protein and/or nucleic acid can be carried out using conventional techniques known in the art.

In one embodiment, however, said value of an inflammation parameter does not involve an establishment of an exact amount of protein/nucleic acid sequence. A mere indication that said protein/nucleic acid sequence is significantly upregulated, or downregulated, may be sufficient.

A value of an inflammation parameter may also be an establishment that a certain protein/RNA sequence is present - or absent - at detectable levels at all. Such value has a boolean character. A value of an inflammation parameter may have yet other characteristics, depending on the parameter involved.

In a preferred embodiment, said inflammation parameter is measured from RNA present in said sample. More preferably, said RNA comprises mRNA. mRNA is particularly indicative for an expression profile of cells of said sample.

More preferably, said inflammation parameter comprises a relative amount of RNA of at least one gene involved in inflammation. Said RNA preferably comprises mRNA. According to the invention, an enhanced or lowered amount of at least one RNA sequence encoding an inflammation parameter is indicative for a risk of infarction related to atherosclerosis. It is preferred to determine RNA amounts, because measuring plasma protein levels is hampered by the half-life of protein and by the requirement of separate and appropriate assays for each protein. Moreover plasma proteins may be originating from several sites, such as from endothelium, from inflamed endothelium, from leukocytes and/or from secretion of proteins into the plasma in particular organs. If an overall result of such different sources of protein is measured, a less reliable result may be obtained as compared to RNA. These obstacles are overcome by measuring (relative) RNA amounts. In a preferred embodiment, relative RNA expression in leukocytes is measured.

According to the invention, an inflammation parameter is particularly indicative for infarction related to atherosclerosis if said parameter is present in, or derived from, a cell or derivative thereof present in said sample. Preferably, a multiplex ligation-dependent probe amplification technique is used in order to determine relative amounts of several RNA sequences (outlined in (19), incorporated herein by reference). With this technique it is possible to quantify relative amounts of multiple RNA molecules simultaneously. No separate assays need to be performed, while a reliable indication of a risk of disease can be obtained. However, other methods known in the art may as well be used to determine the presence and/or amount of certain RNA sequences, such as RT-PCR, Northern blotting, etc.

A parameter of the invention preferably comprises an amount of RNA of at least one inflammatory gene in a sample. However, in case an amount of mRNA is essentially proportional to the amount of corresponding protein in a sample, it is sufficient to measure the amount of protein. An amount of mRNA is essentially proportional to the amount of corresponding protein present in a sample if a certain change in amount of said mRNA results in an essentially corresponding change in amount of said protein in said sample. Preferably, said change in amount of said RNA is the principal, most preferably only, source of change of amount of said protein in said sample. If a protein level is either not influenced by other sources, or influenced to the same extent by other sources in healthy and diseased individuals, essentially independent of a risk of disease, said protein level may be essentially proportional to the amount of mRNA. The terms "amount of protein" and "protein level" are used herein interchangeable. Once a proteinaceous molecule has been identified as a suitable parameter for disease, it can be detected and/or quantified in a sample using known methods in the art, such as ELISA or affinity chromatography. A parameter of the invention may thus comprise the presence and/or amount of a certain proteinaceous molecule.

In a preferred embodiment, a method of the invention is provided wherein said inflammation parameter comprises a relative amount of expression products of at least two genes involved in inflammation. Said expression products preferably comprise RNA, more preferably mRNA. Presence and/or amount of certain combinations of expression products provides a more precise diagnosis of a risk of infarction related to atherosclerosis. A combination for instance provides a more reliable diagnosis if another inflammatory event such as infection or autoimmune disease is present in a subject. Such other inflammatory event may influence an expression level of a certain inflammation marker as well. Said other inflammatory event is however unlikely to alter expression levels of more than one inflammation marker in the same way as compared to the influence of a risk of an infarction related to atherosclerosis upon said expression levels. In order to more efficiently rule out such other inflammation events, it is preferred to determine whether a subject has an expression level of a certain combination of inflammation expression products that is indicative for a risk of an infarction related to atherosclerosis. In one embodiment a parameter of the invention comprises the presence/amount of a certain combination of at least two proteinaceous molecules. Preferably, a combination of between two and four expression products is used as a parameter of the invention. With a method of the present invention, combinations that are indicative for said risk can be identified.

In one embodiment, a relative amount of at least two nucleic acid sequences comprising at least a functional part of at least two genes involved in inflammation is used as a parameter of the invention. Preferably, an amount of at least two nucleic acids comprising at least a functional part of at least two genes involved in inflammation is used. Said nucleic acid preferably comprises mRNA.

By at least a functional part of a gene is meant herein a part of said gene, at least 30 base pairs long, preferably at least 200 base pairs long, comprising at least one expression characteristic (in kind not necessarily in amount) as said gene. A functional part of a gene may for instance consist of the coding sequence of a protein, without additional sequences such as a promoter or enhancer.

Table 3 and 5 comprise genes which are at least in part differently expressed in subjects at risk of undergoing an infarction related to atherosclerosis as compared to healthy individuals (such genes are indicated as Inflammatory Marker with Adjusted OR that is significantly higher or lower than 1). Such differently expressed genes are indicative for said risk. Hence, a (relative) amount of such expression product in a sample can be used as a parameter of the invention.

One embodiment provides a method of the invention wherein said inflammation parameter comprises a relative amount of at least one expression product encoded by the gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A. Yet another-embodiment provides a method of the invention wherein said inflammation parameter comprises a relative amount of a nucleic acid comprising at least a functional part of the gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A. Said nucleic acid preferably comprises RNA, more preferably mRNA. In a more preferred embodiment, a method of the invention is provided wherein said gene comprises MIF, PI9, CCL3, PTPN1, BMI1, PDE4B, GSTP1, NFKB1, IL12A, PDGFB, MYC, IL1B, IL8, NFKBIA, IL15(1) and/or IL1A. In an even more preferred embodiment, said gene comprises MIF, PI9, CCL3, PTPN1, and/or BMI1. Most preferably, said gene comprises MIF and/or PI9.

Preferably, amounts of combinations of such expression products are determined in order to even more accurately estimate said risk.

One embodiment provides a method of the invention wherein the value of said inflammation parameter is compared with a threshold value. Said threshold value may for instance be a mean value of a certain parameter in healthy individuals. Alternatively, said threshold value may be derived from the same subject that is investigated for a risk of infarction related to atherosclerosis. A subject may be diagnosed at different time points. For instance, the value of at least one, but preferably at least five or ten, inflammation parameters may be estimated at the age of 20. The obtained values may be considered to be threshold values. Subsequently, said subject may be investigated routinely at regular time intervals. For instance, said subject may be investigated once in five years, or each year, especially once the age of 40 or 50 has been reached. Measured values can subsequently be compared with the threshold values obtained at the age of 20. If a value of at least one of said parameters appears to be changed significantly as compared with said threshold value, it is indicative for an altered status of said subject, such as a disorder. If a value of at least one inflammation parameter (or of at least one combination of parameters) corresponds to a significant extent to a value of a significantly differently expressed gene, such as differently expressed genes indicated in table 3 and 5, said subject can be considered to be at risk of undergoing an infarction related to atherosclerosis. Early diagnosis of such risk is highly desired since said risk can be minimized by appropriate modifications in lifestyle and preventive therapies. Precautionary measures can be taken at an early stage. A subject can counteract a risk of an infarction related to atherosclerosis by eliminating controllable risk factors. Prevention may comprise lowering cholesterol levels, lowering blood pressure, quitting smoking, losing weight, etcetera. Hence, early diagnosis of a risk of an infarction related to atherosclerosis is very important in order to prevent occurrence of an attack. The invention therefore provides a method for reducing the risk for a subject of undergoing an infarction related to atherosclerosis comprising at least in part eliminating a controllable risk factor of said subject, characterized in that said subject was identified as being at risk for said attack through a method of the invention. Said controllable risk factor preferably comprises lowering cholesterol levels, lowering blood pressure, quitting smoking, and/or losing weight. Cholesterol levels are preferably lowered with a statin, as discussed below. In one embodiment, a method of the invention is provided for determining whether a subject is at risk of undergoing a myocardial infarction or coronary artery disease.

A sample can be any sample derivable from a subject, such as a tissue sample, blood sample, etcetera. Preferably however, said sample is a circulating body fluid sample. A blood sample is preferred, since a blood sample can be easily obtained from a subject, without much inconvenience. Most preferably a whole blood sample is used. According to the invention, inflammation parameters present in whole blood, which are preferably of a cell and/or derivative thereof present in said sample, are indicative for a risk of an infarction related to atherosclerosis. It is not necessary to obtain a (tissue) sample of a site of local inflammation, which would severely hamper an early diagnosis. It is not necessary to use blood serum either, although it is of course possible to use serum. Investigation of a serum sample requires additional procedures for processing whole blood in order to remove insoluble parts. Such procedures are not necessary for determining the value of an inflammation parameter with a method of the invention.

According to the invention, at least one differentially expression gene, such as a differentially expressed gene that is listed in table 3 and 5, is suitable for determining a risk of infarction related to atherosclerosis in an individual. For instance, said expression product can be used in an amplification and/or quantification assay. Common RNA amplification assays for instance comprise RT-PCR. Quantification of RNA can be performed with RT-MLPA (described below). Quantification of protein is for instance possible with Western blotting, (captured) ELISA, etcetera. In one aspect the invention thus provides a use of an expression product of gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A for determining whether a subject is at risk of undergoing an infarction related to atherosclerosis. Preferably, said gene comprises MIF, PI9, CCL3, PTPN1, BMI1, PDE4B, GSTP1, NFKB1, IL12A, PDGFB, MYC, IL1B, IL8, NFKBIA, IL15(1) and/or IL1A. More preferably, said gene comprises MIF, PI9, CCL3, PTPN1, and/or BMI1. Most preferably, said gene comprises MIF and/or PI9.

Once a risk of an infarction related to atherosclerosis has been established, risk factors can be eliminated in order to prevent an attack. Many risk factors such as smoking, obesity, hypertension and hypercholesterolaemia can be diminished by appropriate modifications in life style. Additional medication may be necessary in severe cases. Statins are commonly used in order to lower levels of low-density lipoproteins (LDL). LDL is a major contributor to plaques in artery walls. Administration of statins has been shown to significantly decrease a risk of an infarction related to atherosclerosis, such as myocardial infarction. A statin is therefore suitable for the preparation of a medicament for the treatment of a subject at risk of undergoing an infarction related to atherosclerosis. Hence, once it has been estimated with a method of the invention that a subject is at risk of undergoing an infarction related to atherosclerosis, early treatment with a statin has become possible in order to reduce said risk. The invention therefore provides a method for reducing the risk for a subject of undergoing an infarction related to atherosclerosis comprising providing said subject with a statin, characterized in that said subject was identified as being at risk for said infarction through a method of the invention. In another aspect the invention provides a use of a statin for the preparation of a medicament for the treatment of a subject at risk of undergoing an infarction related to atherosclerosis, characterised in that said subject was identified as being at risk through a method of the invention.

According to the invention, inflammation parameters are indicative for infarction related to atherosclerosis. The presence - or absence - of a significant amount of an expression product of an inflammatory gene can be indicative for said attack. Moreover, the (relative) amount of such expression product may be indicative. Expression of an inflammatory gene may be up- or downregulated in response to an atherosclerotic process occurring somewhere in a subject's body. However, an altered expression of such inflammatory gene may also be a cause of atherosclerosis.

With common methods in the art it is possible to determine whether an inflammation parameter has an adverse effect upon atherosclerosis. For instance, such parameter can be administered to a significant amount of non-human animals and it can be subsequently estimated whether a risk of an infarction related to atherosclerosis is increased. Once a parameter has been found to have an adverse effect upon atherosclerosis, said adverse effect can be counteracted, for instance with a compound capable of at least in part inhibiting said adverse effect of said parameter.

By at least in part inhibiting an adverse effect upon atherosclerosis of an expression product of an inflammatory gene is meant herein that said adverse effect is, at least in part, diminished. Preferably, said adverse effect is no more experienced at measurable levels in presence of said compound. Said compound may be a binding molecule, such as an antibody or functional part, derivative and/or analogue thereof, capable of specifically binding said expression product. Said compound is preferably capable of binding an essential region of said expression product. For instance, it may bind to a part of RNA that is important for translation, such as a coding sequence and/or a promoter. Upon binding of said binding molecule, translation will be seriously hampered. Said binding molecule may as well be a molecule capable of binding at least a functional part of a protein. Binding will, at least in part, prevent an (undesirable) action of such protein. Alternatively, said compound may bind to at least a functional part of a ligand of said protein. A compound capable of at least in part inhibiting an adverse effect upon atherosclerosis of an expression product can be generated using common techniques known in the field. For instance, a binding molecule may be generated using current recombinant expression systems.

The invention thus provides a use of a compound capable of at least in part inhibiting an adverse effect upon atherosclerosis of an expression product of an inflammatory gene for the preparation of a medicament for the treatment of a subject at risk of undergoing an infarction related to atherosclerosis. Said expression product preferably comprises an expression product encoded by the genes MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or ILIA.

Methods for generating a medicament are known in the art. Preferably said medicament comprises a suitable carrier and/or diluent.

A method for at least in part reducing a risk of an infarction related to atherosclerosis of a subject, comprising administering to said subject a medicament of the invention is also herewith provided. A medicament can for instance be administered to a subject orally, by aerosol, as a suppository, etc. For mucosal administration, liposomes are also practically useful.

A functional part of a protein is defined as a part which has the same kind of properties in kind, not necessarily in amount. For instance, a functional part of an antibody has the same binding properties in kind, not necessarily in amount. Said functional part of an antibody for instance comprises a Fab fragment, or single chain variable fragment. A functional derivative of a protein an/or antibody is defined as a protein which has been altered such that the properties of said molecule are essentially the same in kind, not necessarily in amount. A derivative can be provided in many ways, for instance through conservative amino acid substitution.

A person skilled in the art is well able to generate analogous compounds of a protein/antibody. This can for instance be done through screening of a peptide library. Such an analogue has essentially the same properties of said protein/antibody in kind, not necessarily in amount.

The invention also provides a new method for identifying at least one parameter of a disease.

In order to provide a new method for identifying parameters of a disease, the present inventors have developed a screening method based on a multiplex ligation-dependent amplification procedure (MLPA), allowing easy and fast screening of expression product patterns in a sample. (Relative) amounts of RNA sequences of various genes in samples from healthy and diseased have been estimated and compared. According to the present invention, using MLPA technology enables detection of parameters indicative for disease. Preferably, a whole blood sample is used. Expression product patterns obtained with a method of the invention are specific for a certain disorder. Once such expression patterns are known, subjects can be screened for the presence of such expression product pattern in order to determine whether said subject is at risk of suffering from such disorder.

Multiplex ligation-dependent probe amplification (MLPA) has been described for DNA substrates in (19), which is incorporated herein by reference. MLPA is based upon amplification and quantification of probes added to a sample, instead of amplification/quantification of sample nucleic acids. The technique involves incubating a nucleic acid sample with at least one MLPA probe. Preferably, a mixture of different MLPA probes is used. Annealed MLPA probes are subsequently amplified, preferably after unbound MLPA probes are washed away, with primers that are specific for said probes. Preferably, one set of primers is used that is specific for all MLPA probes. This is possible if all MLPA probes have a certain sequence in common. After amplification of said MLPA probes, their (relative) amount is quantified, preferably by measuring the intensity of fluorescent labels that are specific for each probe.

In a preferred embodiment, each MLPA probe comprises a set of at least two oligonucleotides. Both oligonucleotides are capable of annealing at the same target sequence. Preferably, each oligonucleotide is capable of annealing to a distinct part of said target sequence. In a particular embodiment one oligonucleotide of each set contains stuffer DNA of variable length (preferably 19-370 bp), and is most preferably generated after cloning in a series of specially created M13 vectors.

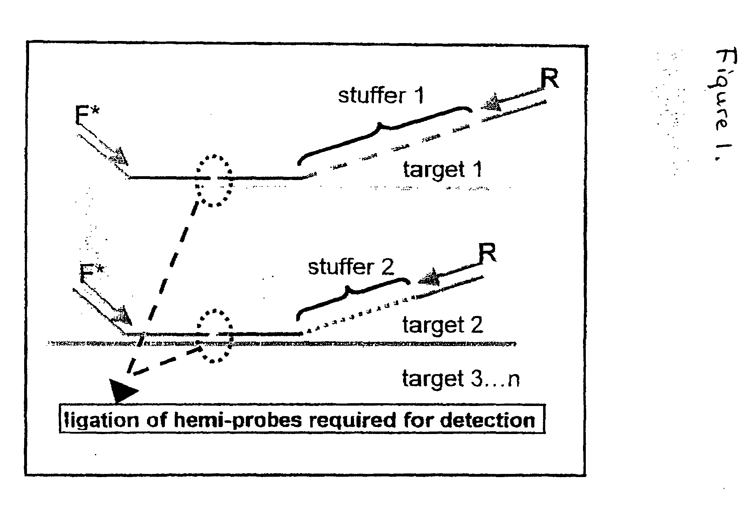

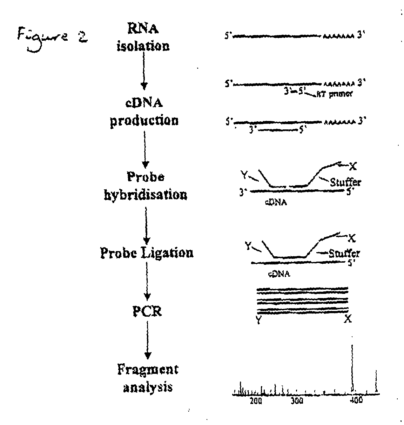

After said oligonucleotides are annealed, they are ligated (preferably around 60°C by a heat-stable ligase). All MLPA probe derived DNA fragments thus obtained, comprising at least two ligated oligonucleotides, contain essentially identical flags at their 5'- and 3'- ends. Subsequently, said fragments comprising ligated oligonucleotides are amplified with primers specific for said probes. Preferably, a single primer pair is used. Afterwards, the amount of DNA fragments derived from said probes can be accurately quantified, for instance on a capillary sequencer. The principle of MLPA is diagrammed in Figure 1.

According to the present invention, suitable parameters for disease can be obtained if MLPA is performed upon nucleic acid derived from a sample of a subject. Preferably said sample comprises a whole blood sample. More preferably, MLPA is performed upon RNA derived from a whole blood sample. Expression profiles of RNA, preferably mRNA, often appear to provide a more reliable insight into parameters specific for disease than other methods known in the art such as detection of soluble protein in a blood sample. Soluble proteins can be derived from several different sources, such as organs (for instance the liver) which secrete proteins into the blood stream, whereas RNA present in whole blood is indicative for leukocyte expression. Without being bound by theory, it is thought that especially expression patterns in macrophages are indicative for disease, or a risk of disease, even if such disease is present elsewhere in the body.

To make the MLPA technique particularly suitable to detect RNA transcripts, an RT-step with gene-specific primers is preferably added prior to the annealing stage. Hemi-probes are preferably designed to span exon boundaries, precluding the detection of potential contaminating genomic DNA.

In order to reliably monitor changes in transcription of target genes, two requirements are preferably met by a technique containing a PCR stage. First, the signal strength and ratios should preferably not be influenced by the number of PCR cycles. Second, even large changes in expression of a certain gene should preferably not influence the signals of the other probes. RT-MLPA is well suited to track changes in expression of target genes.

The invention therefore provides a method for quantifying at least one target nucleic acid in a sample, comprising incubating said sample with at least one probe capable of annealing to said target nucleic acid; amplifying annealed probes; and quantifying said probes, characterized in that said sample comprises a whole blood sample. The invention also provides a method for quantifying at least one target nucleic acid in a sample, comprising incubating said sample with at least one set of at least two oligonucleotides, said at least two oligonucleotides of one set being capable of annealing to (different parts of) a target nucleic acid; ligating oligonucleotides of one set that are bound to a target sequence; amplifying ligated oligonucleotides; and quantifying said oligonucleotides; characterized in that said sample comprises a whole blood sample.

Preferably, said nucleic acid is RNA, more preferably mRNA.

Another embodiment of the invention provides a method for determining whether an expression product is indicative for a disease, comprising determining whether a relative amount of nucleic acid encoding said expression product in a sample of a healthy subject is significantly different from a relative amount of nucleic acid encoding said expression product in a sample of a diseased subject, characterized in that multiplex ligation-dependent probe amplification is performed upon a whole blood sample.

Preferably said nucleic acid comprises RNA, more preferably mRNA, since mRNA is indicative for an expression level. An amount of an RNA sequence can change rapidly in response to altered circumstances, such as a risk or the onset of a disease. Changes of protein levels often cannot be quickly changed, for instance depending on the half-life of said protein.

As explained above, once an expression product has been identified as being indicative for disease, it can be established whether said expression product has an adverse effect upon such disease. In that case a medicament can be prepared comprising a compound capable of at least in part inhibiting said adverse effect. The invention therefore also provides a use of a compound capable of at least in part inhibiting an adverse effect upon a disease of an expression product identified with a method of the invention for the preparation of a medicament for the treatment of a subject suffering from, or at risk of suffering from, said disease.

Also provided is a kit of parts comprising suitable means for performing a method of the present invention. Said suitable means for instance comprise means for estimating presence and/or amount of RNA and/or protein present in a sample. Preferably a kit of parts is suitable for performing a MLPA reaction. With such kit simultaneous quantification of mRNA levels of multiple genes is possible. A relative small amount of whole blood is sufficient. With MLPA it is not necessary to isolate white blood cells from a blood sample. Moreover, MLPA can be applied upon total RNA extracts.

A kit of parts of the invention preferably comprises:
- at least one set of MLPA probes capable of annealing to a nucleic acid encoding MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A;
- suitable means for performing a MLPA reaction.

More preferably, a kit of parts of the invention comprises:
- at least one set of MLPA probes capable of annealing to a nucleic acid encoding MIF, PI9, CCL3, PTPN1, BMI1, PDE4B, GSTP1, NFKB1, IL12A, PDGFB, MYC, IL1B, IL8, NFKBIA and/or IL15(1);
- suitable means for performing a MLPA reaction.

More preferably, a kit of parts of the invention comprises:
- at least one set of MLPA probes capable of annealing to a nucleic acid encoding MIF, PI9, CCL3, PTPN1, and/or BMI1;
- suitable means for performing a MLPA reaction.

Most preferably, a kit of parts of the invention is provided, wherein said kit comprises suitable means for isolation of nucleic acids from whole blood.

The invention is further explained in the following examples. The examples merely serve to clarify the invention. They do not limit the invention in any way. Many alternative embodiments are within the scope of the present invention.

### Examples

### Example 1

### Methods

Patients and control subjects Cases were men consecutively diagnosed with a first myocardial infarction before the age of 70 between January 1990 and January 1996. Control subjects were men without a history of myocardial infarction who had a minor orthopaedic intervention between January 1990 and May 1996 and had received prophylactic anticoagulation treatment for a short period after this intervention. They were selected from the files of the Leiden Anticoagulation Clinic -which serves the same region as the hospitals in which the cases were recruited - and were frequency matched to the patients on 10-year age groups. Only patients seen at least 6 months after the date of the event, or index date, were included in this study to ensure that inflammatory reactions surrounding the cardiac event or orthopedic intervention had subsided. The median time between the index date and blood collection was 2.8 years (range 0.6 years to 6.3 years) for cases and controls alike. RNA samples were available for analyses from 524 cases and 628 controls. Details of this population-based case-control 'Study of Myocardial Infarctions (SMILE)' are described elsewhere [26].

RNA analysis Morning fasting citrated blood samples were drawn from the antecubital vein. Immediately thereafter, small aliquots of 100 µl were added to 900 µl of lysis buffer [20]. The samples were stored at -70°C until further use. RNA was isolated using a silica-based method [20] and analysed by multiplex ligation-dependent probe amplification (MLPA) using a kit developed in collaboration with MRC-Holland for the simultaneous detection of 38 messenger RNA molecules [27]. All samples were tested with the same batch of reagents, and a negative and lipopolysaccharide-stimulated control sample were included on each plate. The final PCR fragments amplified with carboxyfluorescein(FAM)-labeled primers were separated by capillary electrophoresis on a sixteen capillary ABI Prism® 3100 Genetic Analyzer. Peak area and height were processed using GeneScan® analysis software. The levels of mRNA for each gene were expressed as a normalised ratio of the peak area divided by the peak area of a control gene, (i.e., a gene with constant, constitutive expression independent of the atherosclerotic status of the patient), resulting in the relative abundance of mRNA of the genes of interest. Our probe set is listed in Table 1 and contains probes for mRNAs of 35 inflammation and apoptosis related proteins, and three control genes, i.e. B2M, CDKN1A and PARN. PARN and CDKN1A mRNA levels were always within the limits of detection whilst B2M levels were above the upper limit of detection. Therefore peak areas were normalised to PARN and to CDKN1A resulting in similar expression profiles. We have opted to present the results relative to CDKN1A due to its insensitivity to in vitro lipopolysaccharide stimulation of an inflammatory response [27] (Spek et al, unpublished).

Statistics Median values of the relative mRNA levels of cases and control subjects were compared using a Mann-Whitney test. Persons with values above the upper detection limit were assumed to have the highest levels, whereas persons with values below the detection limit were assumed to have the lowest mRNA level for that marker. Odds ratios (OR) were calculated as an estimate of the relative risk for myocardial infarction. Quartiles were defined on the basis of the mRNA distribution among control subjects. The lowest quartile was used as a reference category for calculating odds ratios. 95% confidence levels (CI95) were calculated according to the method by Woolf [28] or were derived from the standard errors calculated by the logistic model. For variables with more than a quarter of the readings below the lower limit of detection, odds ratios were calculated for persons with detectable levels compared to those with non-detectable levels. About three-quarters of the samples had readings for PTP4A2 above the upper detection limit; therefore the relative risk of myocardial infarction was estimated by calculating the odds ratio for persons with levels above the upper detection limit compared to those within the detection limits. Odds ratios were adjusted for traditional cardiovascular risk factors such as age, smoking, hypertension, hypercholesterolemia, diabetes, body mass index (BMI), alcohol use and quartile of CRP by using unconditional logistic regression. The odds ratios were further adjusted for time between the index date and blood sampling. Since 185 cases stopped smoking in the interval between the time of their myocardial infarction and blood withdrawal, and smoking may influence some of the inflammatory markers, odds ratios adjusted for age were also calculated restricted to men who were non-smokers both before and after the index date.

### Results

It was possible to isolate RNA and perform MLPA analysis on 524 cases and 628 control subjects that fit the criteria required for this study. Mean age was 57 (range 27-75) years for cases and controls alike. Out of the cases 62% were smokers compared to 33% of control subjects (Table 2).

Median leucocyte levels (normalised to CDKN1A) of most inflammatory mRNAs were higher in men who had a myocardial infarction compared to control subjects (Table 1). These differences were further evaluated with an analysis based on mRNA levels after stratification in quartiles (Table 3) and most resulted in elevated odds ratios that were not influenced much by adjustment for traditional risk factors and plasma levels of C-reactive protein. The highest adjusted odds ratios were observed for the highest versus lowest quartiles of mRNA levels of MIF, PI9, CCL3, PTPN1 and BMI1, and were between 2 and 3. PDE4B, GSTP1, NFKB1, IL12A, PDGFB, MYC, IL1B, IL8, NFKB1A and IL15(1) had odds ratios between 1.5 and 1.9 after adjustment, whilst LTA, IFNG, IL1RN, TNFRSFIA, IL18 and CCL4 had adjusted odds ratios of 1.3 or 1.4. THBS1 and PARN were not associated with myocardial infarction (ORadj 0.9, and 1.0 respectively). The full data in quartiles are shown in Table 4 for a selection of markers that show increasing odds ratios with increasing levels of mRNA.

Odds ratios for markers with more than a quarter of the readings below detection levels are shown in Table 5. In this comparison of detectable versus undetectable levels, IL15(2), MCP-2 and IL2 were associated with an approximately two-fold increase in odds ratio. IL12B had an adjusted odds ratio of 1.6 and IL6, TNF, IL4(2), and IL10 had a somewhat lower adjusted odds ratio of 1.4. Too few samples had detectable levels of IL13 and TF mRNA (9 and 15 samples respectively). MCP-1 and NFKB2 had an odds ratio of 1.2 (CI95 0.9-1.6 for both) and IL4 was not associated with myocardial infarction. IL1A was peculiar because it gave an odds ratio below one indicating a protective effect (OR 0.6, CI95 0.5-0.8). Hence, downregulation of IL1A is indicative for a risk of an infarction related to atherosclerosis.

Most of the readings for PTP4A2 were above the upper detection limit. The ORadj for persons with levels above the upper detection limit compared to those with levels within the detection limits was 1.7 (CI95 1.2-2.3).

Few of the crude odds ratios were changed by adjustment for cardiovascular risk factors, and only seven mRNA markers (LTA, MIF, IL1B, IL1RN, MYC, NFKB1 and CCL4) showed differences between the crude and adjusted odds ratios, with slightly lower adjusted odds ratios in most of these. Multivariate analysis indicated that smoking accounted for most of these differences (analysis not shown). A stratified analysis showed that the inflammatory markers have elevated odds ratios even in non-smokers, and in fact in most cases the odds ratios were higher in men who did not smoke at or after the index date. This is particularly striking for the highest compared to the lowest quartile of mRNA levels of MIF with an age-adjusted odds ratio of 5.2 (95CI 2.9-9.5) in non-smokers.

Time since the index date did not change the adjusted odds ratios for the highest quartiles, except for those of MIF, PI9 and PTPN1 which increased to 3.5 (95CI 2.3-5.2), 2.9 (95CI 2.0-4.2), and 2.6 (95CI 1.7-3.7) respectively.

### Discussion

Patients with a history of myocardial infarction have a different mRNA signature for inflammatory markers than healthy control subjects. Increased mRNA levels of MIF, PI9, CCL3, PTPN1, BMI1, and IL15(2) were found to be associated with myocardial infarction with odds ratios in the range of 2-3 for patients compared to control subjects. Elevated mRNA levels of PDGFB, IL12A, IL12B, MYC, NFKB1, GSTP1, IL18, IL15(2), IL1B, IL1RN, IL8, NFKBIA, PDE4B, MCP-2 and IL2 all yielded odds ratios for myocardial infarction varying between 1.5 and 2.

All proteins encoded by the mRNAs that were associated with myocardial infarction play important roles in inflammation and apoptosis. For example, CCL3 is involved in the recruitment and activation of polymorphonuclear leucocytes in acute inflammation; NFKB1 and NFKB2 belong to the proinflammatory NFKB family of transcription factors that are inhibited by NFKBIA; the protein tyrosine phosphatases are important in regulating signal transduction, neoplastic transformation and the mitotic cycle. BMI1 is involved in transcriptional silencing of certain genes relating to cell proliferation and senescence, and IL15 is involved in T-cell and NK-cell growth and proliferation.

The absence of a noticeable effect of adjustment shows that these associations are not brought about by traditional risk factors. The only cardiovascular risk factor that had an effect on some of the odds ratios (LTA, MIF, IL1B, IL1RN, MYC, NFKB1 and CCL4) was smoking. This finding is not surprising since it is generally accepted that smoking enhances vascular inflammation, and some systemic inflammatory markers, including CRP, have been found to be higher in smokers than former or never smokers. The results of the stratified analysis on non-smokers show that the inflammatory markers have an effect themselves, and that smoking masked some of this effect.

Adjustment for levels of C-reactive protein did not change the odds ratios. This underlines that in cardiovascular disease inflammation plays a role in three compartments. The first is in the inflamed vascular wall where many inflammatory cells are known to accumulate. The second is in the liver where acute phase reactants such as C-reactive protein are synthesized. The results from our study clearly show that there is a third compartment with a signature of inflammation in circulating leucocytes.

### Example 2

Treatment with an anti-CD14 monoclonal antibody delays and inhibits lipopolysaccharide induced gene expression in humans in vivo.

Lipopolysaccharide (LPS), also called endotoxin, is a component of the outer membrane of Gram-negative bacteria that activates the innate immune system [1-3]. Although this activation is important for an adequate host defense against Gram-negative bacteria, under certain conditions the uncontrolled activation of the immune system can result in life-threatening clinical symptoms of sepsis [4]. The initial step in cellular activation is the interaction of LPS with LPS binding protein (LBP) [5] with the subsequent transfer of LPS to the cell surface receptor CD14 present on different cell types, including monocytes, macrophages and granulocytes [6]. Alternatively, the LPS-LBP complex interacts with soluble CD14 found in plasma resulting in the activation of CD14 negative cells, like for instance endothelial cells [7,8].

Recognition of LPS by CD14 triggers signal transduction through Toll-like receptor 4 [9,10], eventually leading to activation of nuclear factor (NF)κB [11] and production of a series of pro- and anti-inflammatory mediators, like tumor necrosis factor (TNF)-α, interleukin (IL)-6, IL-8, IL-1 receptor antagonist (IL-1Ra), and macrophage inflammatory proteins (MIP)-1α and -1β [12-14].

The CD 14 dependence of LPS induced signaling responses provides a rationale for blocking CD 14 function in order to reduce the consequences of Gram-negative septic shock. Indeed, both animal and human studies have shown that blocking CD14 almost completely inhibits the inflammatory response evoked by LPS or Gram-negative bacteria [13,15-17]. In accordance, treatment with the anti-CD14 antibody IC14 during human endotoxemia strongly inhibited LPS-induced pro-inflammatory cytokine release, whereas the release of the anti-inflammatory cytokines soluble TNF receptor type 1 (sTNFRl) and IL-1Ra was only delayed [13]. In addition, IC14 treatment also inhibited LPS-induced IL-8, MCP-1 and MIP-1β chemokine production, while LPS-induced MIP-1α levels were neither inhibited nor delayed [18].

The prominent role of CD14 in LPS-induced cellular signaling and the promising characteristics of IC14 in reducing clinical symptoms and inhibiting inflammatory responses during human endotoxemia warrants studying the molecular effects of IC14 in more detail. Therefore, in this example, we employed multiplex ligation-dependent probe amplification (MLPA; [19], a recently developed technique) for the simultaneous relative quantification of about 30 different mRNA's, to determine the effects of IC14 on LPS induced gene expression in human volunteers.

### Methods

Study design - The present study was performed simultaneously with studies on the effect of IC14 on LPS-induced cytokine and chemokine release of which the results have been published previously [13,18]. Sixteen healthy male volunteers (mean age, 23 years, range 20-33 years) received an intravenous injection of Escherichia coli LPS (Lot G, VSP, Rockville, MD) at a dose of 4 ng/kg body weight. The volunteers were all in good health as monitored by medical history, physical examination, and routine laboratory examination. All individuals were admitted to the Clinical Research Unit (Academic Medical Center, University of Amsterdam) and remained under the supervision of trained medical staff during the whole period of the study. Blood was collected from the antecubital vein immediately before LPS injection and at 0.5, 1, 2, 3, 4, 6, 8, and 21 hours thereafter. The Institutional Ethics and Research Committees approved this study, and written informed consent was obtained from all study subjects. The participants did not smoke, use any medication, have any febrile illness in the 30 days preceding the study and had never received monoclonal antibody therapy. The volunteers fasted overnight before LPS injection. Eight of the volunteers received IC14, whereas eight were given placebo starting two hours prior to LPS administration.

IC14, supplied by ICOS (Bothell, Washington), is a recombinant chimeric (murine/human) mAb recognizing human CD14. The murine parent is an Ab designated 28C5 [16,17], which is secreted from Chinese hamster ovary (CHO) cells as an L2H2γ4 Ig. IC14 was isolated from the sterile harvest fluids of CHO cells grown in bioreactors using affinity ion exchange and hydrophobic interaction chromatography. IC14 (1 mg/kg in 150 ml 0.9% (w/v) NaCl) was administered intravenously over a one-hour period through a 0.22 mm low protein-binding filter. The placebo solution, consisting of the dilution fluid was administered in an identical manner.

Leucocyte counts - Leukocyte counts and differentials before and after LPS injection were assessed by a Stekker analyzer (counter STKS, Coulter counter, Bedfordshire, UK).

RNA isolation - Total nucleic acids were isolated from whole blood according to a solid-phase extraction method described by Boom et al [20]. Briefly, 100 µL whole blood was mixed with 900 µL lysis buffer (50 mmol/L Tris-HCl [pH 6.4], 20 mmol/L EDTA, 1.3% [wt/vol] Triton X-100, 5.25 mol/L guanidine thiocyanate). After a phenol-chloroform extraction, 70 µL of activated silica suspension (1 g/mL) was added to the lysis mixture. After washing and drying the silica, nucleic acid was eluted with 50 µL elution buffer and stored at -70°C.

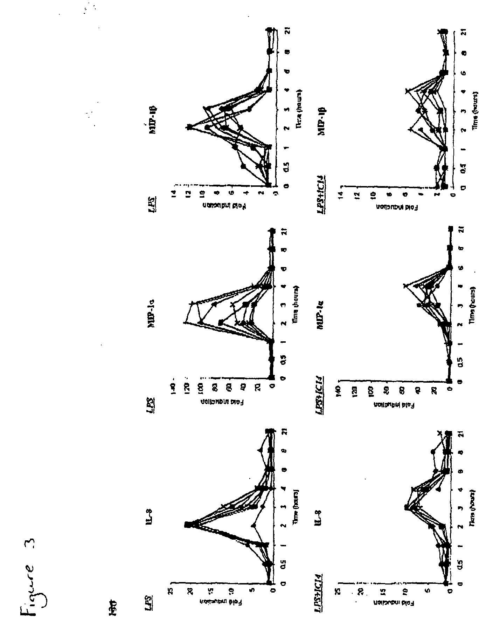

Multiplex ligation-dependent probe amplification (MLPA) - MLPA (figure 2) was performed essentially as described by Schouten et al. [19] to analyse mRNA expression of a set of proteins involved in inflammation (table 6). Total RNA is used to prepare cDNA using gene-specific RT primers. Relative amounts of each cDNA are determined by MLPA using probes that are cDNA specific as the probe binding sites overlap an exon boundary. Briefly, 10-50 ng of total RNA in a 5 ml reaction mixture containing: 50 mM Tris [pH 8.5], 75 mM KCl, 3 mM MgCl2, 10 mM DTT, 2.5 nmol of each dNTP and 500 fmol of each gene specific RT primer (www.mrc-holland.com) was, after heating for 1 minute at 80°C and incubation at 45°C for 5 minutes, incubated at 37°C for 15 minutes with 6 U/mL MMLV reverse transcriptase (Promega, Leiden, The Netherlands). After heat inactivation of the reverse transcriptase by incubation at 98°C for two minutes, 1.5 mL of probe mix containing 0.5-4 fmol of each individual MLPA probe oligonucleotide (www.mrc-holland.com) was added together with 1.5 mL MLPA hybridization buffer (1500 mM KCl, 300 mM Tris [pH 8.5], 1 mM EDTA). Next, the hybridization mixture was heated to 95°C for 1 minute and incubated at 60°C for 16 hours. Ligation of the hybridized probes was performed at 54°C for 15 minutes by addition of 31ml ligation mixture (2.6 mM MgCl2, 5 mM Tris [pH 8.5], 0.013% (vol/vol) nonionic detergents, 0.2 mM NAD, 1 U Ligase-65 (MRC Holland, Amsterdam, The Netherlands)). Finally, after the inactivation of Ligase-65 by incubating the samples at 98°C for 5 minutes, the ligation products were PCR amplified.

PCR - 10 mL of ligation mixture was incubated in a 40 mL reaction volume containing 15 mM Tris-HCl [pH 8.3], 50 mM KCl, 1.5 mM MgCl2 and 0.15% w/v non-ionic detergents. Whilst at 60°C, a mix containing 2.5 nmol of each dNTP, 10 pmol of each of the two PCR primers (D4-labeled pr 1: 5'-GGGTTCCCTAAGGGTTGGA-3' and pr 2: 5'-GTGCCAGCAAGATC CAATCTAGA-3') and 2.5 units SALSA polymerase (MRC-Holland) was added. After 1 minute heating at 95°C, 32 PCR cycles were carried out, each cycle consisting of 30 sec denaturation at 95°C, 30 seconds annealing at 60°C and 1 minute extension at 72°C.

Quantification of relative abundance - PCR fragments amplified with a D4-labeled primer (Research Genetics) were analyzed on a Beckman CEQ2000 eight capillary electrophoresis system. Peak area and size data were processed using in-house software and imported into a spreadsheet program. The peak areas of the different genes were divided by the peak area of □-2-microglobulin (a gene known to be LPS unresponsive [21] resulting in the relative abundance of the genes of interest. The absolute amount of ligation products obtained for several (abundant) mRNAs during the MLPA reaction was reduced by inclusion of competitor oligonucleotides for these probes in the MLPA probe-mix (www.mrc-holland.com). The relative amounts of probe signals as shown in Table 6 do not reflect the absolute amounts of these mRNAs. To prevent this, and differences in basal levels to obscure the results, we therefore chose to indicate fold induction (relative abundance at tx /relative abundance at t0).

### Results

### Leukocyte counts

LPS administration induced profound changes in peripheral blood cells fractions as reported in part previously [13,18]. Table 7 summarizes leukocyte counts and differentials before and after LPS injection, and the influence of IC14 treatment hereon, for LPS responsive cells (neutrophils and monocytes).

### Chemokine gene expression

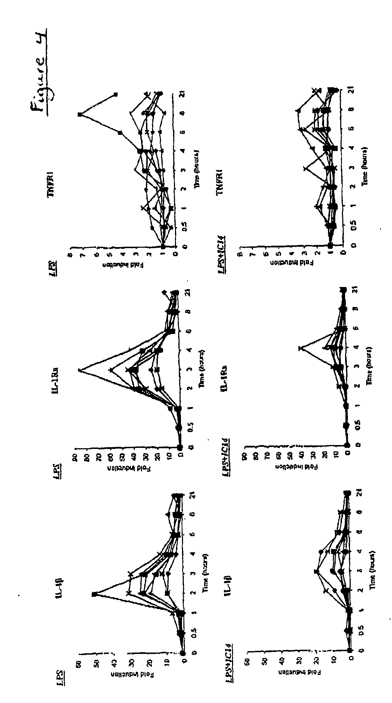

Since chemokines are important pro-inflammatory mediators, we determined gene expression of the chemokines IL-8, MIP-1α, MIP-1β, MCP-1 and MCP-2 in human volunteers challenged with LPS. As evident from figure 3, IL-8 mRNA levels increased approximately 20-fold after LPS injection, peaking after 2 hours. MIP-16 mRNA levels rose 7-fold after LPS administration, reaching peak levels after 2 to 3 hours, whereas the number of MIP-1α mRNA molecules increased about 60-fold with peak levels also after two to three hours (figure 3). MCP-1 and MCP-2 mRNA remained below the detection limit during the whole time frame of the experiment. IC14 treatment delayed and reduced chemokine mRNA levels but did not completely abolish transcription of the chemokine genes. As is evident in figure 3, due to IC14 treatment chemokine mRNA levels decreased approximately 2-fold, whereas maximal levels were delayed by one hour.

### Cytokine gene expression

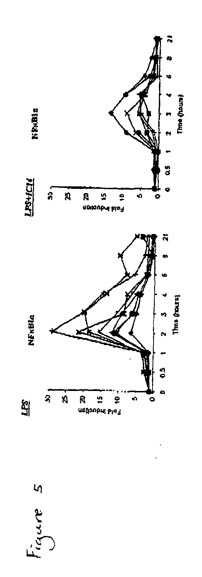

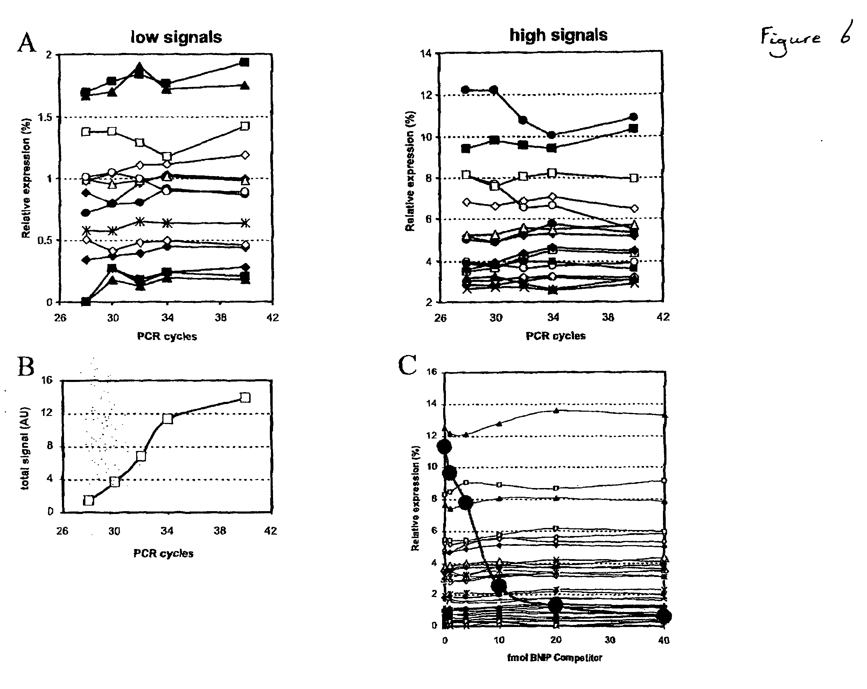

LPS induced detectable gene expression of IL-1Ra, IL-1β and sTNFR1, whereas all other cytokine mRNAs from table 6 (IL-1α, IL-2, IL-4, IL-6, IL-12α, IL-13, IL-15, IFNγ and TNFα) remained below the detection limit at all time points. As shown in figure 4, IL-1β mRNA levels increased about 25-fold after LPS injection with peak levels after 2-3 hours. IL-1Ra levels had increased 40-fold at 3 hours after LPS injection (fig 4). TNFR1 mRNA levels were detectable at baseline and did not change due to the LPS challenge (fig 4). IC14 treatment reduced the induction of IL-1B and IL-1Ra mRNA levels about 8- or 5-fold, respectively, and delayed peak mRNA levels by 1 hour (figure 4).

### NFκB gene expression

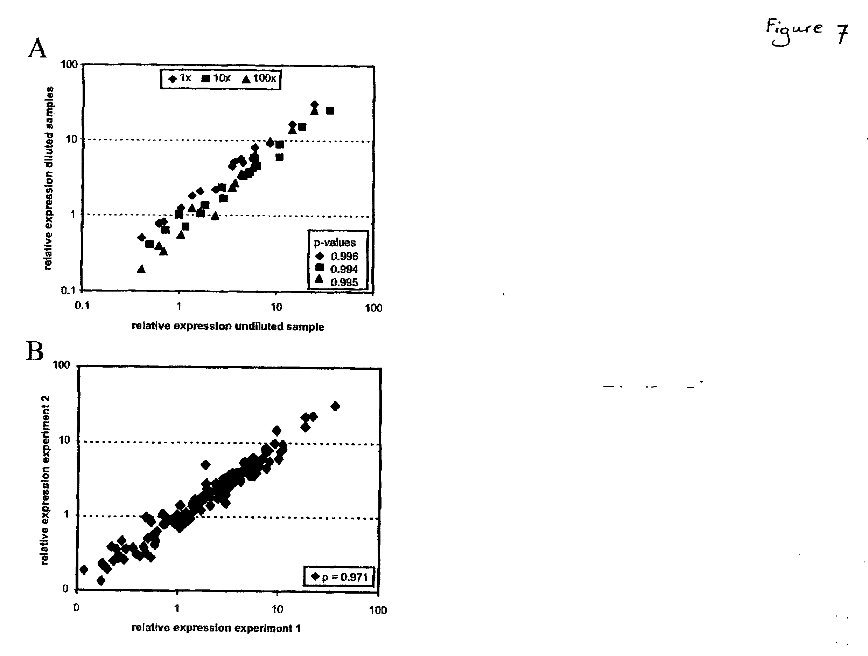

Since NFκB is the key signal transduction molecule involved in inflammation, we determined gene expression of NFκB family members IκBα, p50, p49/p100 and p65 (c-rel). As shown in figure 5, LPS induced an approximately 15-fold increase in IκBα mRNA levels that peaked two hours after the injection of LPS. p50 mRNA levels were detectable but were LPS unresponsive (data not shown), whereas p65 and p49/p100 were below the detection limit during the observation period. Treatment with the CD14 blocking antibody IC14 reduced LPS induced IκBα levels about 3-fold (5-fold induction) and shifted the maximal LPS response from 2 to 3 hours after the injection (figure 5).

### Discussion

This example clearly shows that the CD14-blocking antibody, IC14, diminished and delayed gene expression in peripheral blood cells of human volunteers challenged with LPS. LPS-induced mRNA levels of chemokines, cytokines and NFκB family members were reduced but certainly not completely abolished by IC14 treatment. In the volunteers treated with IC14, LPS still induced IL-8, IL-16 and IL-1Ra mRNA production about 10-fold, whereas MIP-1α levels were even induced 40-fold. In addition, MIP-1β and IκBα expression was also induced in the IC14 treated volunteers, albeit, to a lesser extent. Quite remarkably, LPS-induced peak levels of gene expression were reached 1 hour later in the IC14 treated than in the placebo treated volunteers.

A direct and an indirect pathway are generally believed to be involved in the onset of the innate immune response after a LPS challenge. In the direct pathway, LPS complexes with soluble LBP and is subsequently transferred to membrane bound CD14 expressed on different cell types. Alternatively, the LPS-LBP complex may interact with soluble CD14, that is present in blood in high concentrations (2-6 mg/ml [22]), resulting in the activation of CD14 negative cells, such as endothelial cells. The resulting response leads to the secretion of inflammatory proteins into the bloodstream. Blocking CD14 during endotoxemia showed a differential effect on the production of these inflammatory proteins. TNF-α, IL-6 and IL-8 levels are almost completely inhibited [13,18], whereas MIP-1β and MCP-1 levels are reduced only 3-fold [18]. In addition, IC14 delays (but does not inhibit) sTNFR1 and IL-1Ra secretion and has no effect on MIP-1α plasma levels [13,18]. However, whether IC14 affects the secretion of inflammatory proteins by peripheral blood cells or by non-circulating cells such as tissue macrophages, endothelial cells or monocytes attached to the endothelium cannot be concluded from such experiments. For this reason, we performed gene expression analysis in peripheral blood cells derived from human volunteers challenged with LPS. These peripheral blood cell mRNA data do not correspond very well with plasma protein levels (see table 8). For instance, IC14 inhibits and delays both MIP-1α and IL-1Ra mRNA induction in peripheral blood cells, whereas IL-8 mRNA induction is reduced 2-fold only.

Moreover, in the plasma of individuals challenged with LPS, MIP-1α levels increase from 0 to 0.5 ng/ml, whereas MIP-1β increases even to 25 ng/ml [18]. Again, our peripheral blood cell mRNA levels do not correspond with these plasma changes. MIP-1α mRNA expression is induced about 60-fold and MIP-1β only about 7-fold. Additionally, IL-1β mRNA is rapidly induced by LPS in peripheral blood cells, whereas in plasma no or very low levels of IL-1β can be detected [23,24]. Hence, a discrepancy between plasma protein levels and peripheral blood cell mRNA production appears to be present.

It is known that due to regulation of transcription and/or translation mRNA levels not always exactly reflect amounts of proteins produced. Therefore, comparisons of mRNA levels to protein level for individual genes should be interpreted cautiously. In this example we analysed multiple genes which all show a clear discrepancy between peripheral blood cell mRNA expression levels and plasma protein levels (summarized in table 8).

We employed a new technique, called MLPA, to simultaneously determine the relative abundance of 24 genes involved in inflammation during endotoxemia. This technique has proved to be able to detect one-copy number changes of chromosomal DNA sequences [19]. The results described in this paper, indicate the usefulness of this technique for relative quantification of up to 40 different mRNAs in a single reaction using very small amounts of sample RNA (equivalent to 10µl whole blood). The reliability of this experiment, in which mRNA expression levels are expressed relative to β-2-microglobulin mRNA levels is supported by the fact that mRNA levels of ferritin light chain, a known LPS unresponsive gene [21], were constant during the time frame of the experiment (data not shown). Furthermore, LPS induced a rapid increase in monocyte TF expression (data not shown) that are in concordance with previously reported data by Franco et al [25] using a specific TF NASBA.

### Example 3

### Expression profiling via novel multiplex assay allows rapid assessment of gene regulation in defined signaling pathways

Here we describe application of a novel format of the MLPA technique, modified to quantify gene transcripts over a wide expression range. The new procedure was tested on mRNAs that regulate two defined biological processes. The first set of target genes plays a central role in the response of cells to inflammatory conditions. The second probe set is designed to comprehensively monitor genes involved in direct regulation of apoptosis. Both these target gene sets are intensely studied in fundamental and applied research. Basic conditions are defined for high throughput relative quantification of these mRNAs in total RNA samples derived from human cells. The two probe sets were able to reliably measure expression profiles and to detect strong induction of transcription in two relevant model systems.

### EXPERIMENTAL PROTOCOLS

### Reagents and cells

### Cell lines Jurkat T cells and U2OS osteosarcoma cells were cultured in IMDM plus 10% fetal calf serum (FCS) supplemented with penicillin and streptomycin.

Healthy volunteers and patients Blood was collected from the antecubital vein of healthy volunteers or from B cell chronic lymphocytic leukemia (B-CLL) patients using heparin (LEO Pharmaceutical, Weesp, The Netherlands; final concentration, 10 U/mL) or EDTA as anticoagulant. CD8+ T cells from peripheral blood and naive tonsil B cells were obtained as described (29, 30). Human blood samples were obtained upon informed consent.

Whole blood stimulation Stimulation of whole blood with ligands for TLR2, 3, 4, 7 or 9 was performed essentially as described previously (23). Heparinized whole blood (0.5 ml) was diluted with an equal volume of RPMI-1640 (GibcoBRL, Invitrogen, Breda, The Netherlands) or RPMI-1640 containing various stimuli and incubated in polypropylene tubes for 2 hours at 37C. Stimuli used were the TLR2 ligand LTA (10 µg/ml final, kindly provided by Dr. T. Hartung, University of Konstanz, Germany, TLR3 ligand poly(I-C) (50 µg/ml final, Sigma, Zwijndrecht, The Netherlands), TLR4 ligand LPS (1 µg/ml final, E. coli LPS 0111:B4, Sigma), TLR7 ligand imiquimod (1 µM final, Sequoia Research Products Ltd, Oxford, UK), TLR9 ligand CpG-2006 oligodeoxynucleotides or control nonCpG-2006flip (10 µM final, Eurogentec, Seraing, Belgium). After incubation, cells were collected and processed for RNA isolation.

RNA isolation - Total nucleic acids were isolated from whole blood according to a solid-phase extraction method as described (20). Briefly, 100 µl whole blood was mixed with 900 µl lysis buffer (50 mM Tris-HCl [pH 6.4], 20 mM EDTA, 1.3% [wt/vol] Triton X-100, 5.25 M guanidine thiocyanate). Next, 50 µl of activated silica suspension (1 µg/ml) was added to the lysis mixture. After washing and drying the silica, nucleic acid was eluted with 50 µl elution buffer and stored at 70°C. Alternatively, for T ands B cell samples and cell lines, Trizol reagent (Gibco) was used to isolate total RNA according to the manufacturer's instructions. Commercial RNA samples from prostate cells were obtained from Clontech (Erembodegem, Belgium).

### MLPA Probes and oligonucleotides.

Each MLPA probe consists of one short synthetic oligonucleotide (Biolegio, Malden, The Netherlands) and one phage M13 derived long probe oligonucleotide. Preparation of the M13 derived MLPA probe oligonucleotides has been described (19). All probes used in this study were designed to detect cDNA, and are described in detail in Table 10. In order to avoid detection of contaminating DNA fragments, all target sequences have an exon boundary close to the probe ligation site. For each probe target sequence a specific Reverse Transcription primer was designed that is complementary to the RNA sequence immediately downstream of the probe target sequence. Tm of all RT primers was between 55 and 60°C. Sequence information available from the public databases was used.

### MLPA reaction

MLPA reactions were performed in 200 µl tubes in a thermocycler with heated lid. Total RNA samples were mixed with Reverse Transcriptase (RT) buffer (Final concentration 50 mM Tris- HCl pH 8.5; 75 mM KCl; 3 mM MgCl2, 10 mM DTT), dNTPs (1.25 nmol) and 500 fmol of each probe specific RT primer in a final volume of 5 µl. After heating to 80°C for 1 min and incubation at 45°C for 5 min, 30 units (0.5 µl) MMLV Reverse Transcriptase (Promega, Leiden, The Netherlands) was added and samples were incubated for 15 min at 37°C. After heating 1 min at 98°C, 1.5 µl salt solution (1500 mM KCl, 300 mM Tris-HCl pH 8.5, 1 mM EDTA) mixed with 1.5 µl probe mix (1-4 fmol of each synthetic oligonucleotide and M13-derived oligonucleotide in TE) was added. Samples were heated for 1 min at 95°C and then incubated for 16 h at 60°C. Ligation of annealed oligonucleotides was performed at 54°C by diluting the samples to 40 µl with dilution buffer (2.6 mM MgCl2, 5 mM Tris-HCl pH 8.5; 0.013 % non-ionic detergents) containing 0.2 mM NAD and one unit Ligase-65 and incubation for 15 min. The ligase enzyme was inactivated by heating at 98°C for 5 min and ligation products were amplified by PCR. For most experiments, 10 µl of the ligation reaction was added to 30 µl PCR buffer. While at 60°C, 10 µl of a buffered solution containing the PCR primers (10 pmol), dNTPs (2.5 nmol) and 2.5 units Taq polymerase were added. PCR was for 33 cycles (30 s at 95°C; 30 s at 60°C and 1 min at 72°C). Samples amplified with one unlabeled and one FAM labeled primer were analyzed on an Applied Biosystems 3100 capillary sequencer (Applied Biosystems, Warrington, UK). Samples amplified with one unlabeled and one D4-labeled primer (Research Genetics) were analysed using a Beckman CEQ2000 eight capillary electrophoresis system (Beckman Coulter, Mijdrecht, The Netherlands).

### Data Analysis

After the PCR stage, aliquots of samples were mixed with GeneScan-500 ROX size standards and run on an ABI 3100 capillary sequencer in GeneScan mode. Data were analyzed with GeneScan and Genotyper software packages (ABI), successively. Category tables containing the area for each assigned peak (scored in arbitrary units) were compiled in Genotyper and exported for further analysis with Microsoft Excel spreadsheet software. Alternatively, after the PCR stage samples were mixed with D1-labelled CEQ DNA 600 size standards and analyzed on a Beckman CEQ2000 in formamide. Data was analyzed with the CEQ2000 fragment analysis software and directly exported and analyzed with Microsoft Excel. In general, for the inflammation probe set data, results from all target genes were calculated relative to the signal of ß2- microglobulin. In the case of the apoptosis probe set, the sum of all peak data was set at 100% to correct for fluctuations in total signal between samples, and individual peaks were calculated relative to the 100% value. For the experiment with TLR ligands, all data were first calculated relative to the ß2M signal, and subsequently expressed as values relative to the signal in the untreated medium sample. Signals below the detection limit in medium were assigned a value corresponding to the threshold value for noise cut-off in GeneScan.

### Microarray, RT-PCR

Custom made cDNA microarrays (manuscript in preparation) were probed with fluorescently labeled RNA mixtures derived from peripheral blood lymphocytes, according to standard procedures. Results were calculated using AIDA Array Metrix software (Raytest, Tilburg, The Netherlands). RT-PCR was performed using standard protocols, oligonucleotides used were, for GAPDH: sense GTGAAGGTCGGAGTCAACG and antisense TGAAGACGCCAGTGGACTC.

For A1/Bfl-1 sense ATGACAGACTGTGAATTTGG and antisense TCAACAGTATTGCTTCAGGAG. Scans of 1% agarose gels were analyzed on a Lumi-Imager with Lumi-Analyst (Roche Diagnostics, Almere, The Netherlands)

### Transfection and RNAi

U2OS cells were cultured in 6-well plates and transfected by standard Ca2+-phospate procedure using pcDNA3.1-eGFP as marker for transfected cells. Transfection efficiencies were between 30- 50%. The pSuper plasmid was obtained from R. Agami. Puma sequences targeted were AGACAGGAATCCACGGCTT for Pumi1 and CGAGATGGAGCCCAATTAG for Pumi3, these were incorporated in oligos encoding short hairpins and cloned in the pSuper vector as described (31).

Cells were transfected with 1 µg of plasmid encoding eGFP plus 9 µg of pSuper, or a mixture of 4.5 µg pSuper-Pumil and 4.5 µg pSuper-Pumi3. 16 Hrs after transfection, medium was refreshed and fludarabine (300 µM) was added where required. Cells were incubated for another 24 hrs and analyzed using a Leica DM IRBE microscope. Representative fields were digitally stored via Leica IM50 software.

### RESULTS

The recently described Multiplex ligation-dependent probe amplification (MLPA) technique (19) is a high throughput, PCR based, method to determine the relative copy number of up to 45 DNA sequences in small samples of human DNA. In order to enable sensitive detection of RNA transcripts, the original MLPA technique was modified by introducing an RT-step with gene-specific primers prior to the probe annealing stage. Since the NAD requiring ligase used in MLPA is not capable of ligating DNA probes that are annealed to an RNA strand, the synthesis of cDNA was required. Hemi-probes were designed to span exon boundaries, precluding the detection of potential contaminating genomic DNA. Target genes for the inflammation and apoptosis probe sets are listed in Table 9 (for exact sequence, location and length of the probes used in both probe sets, see Tables 10 and 11. Further details are described elsewhere (19) and in experimental procedures.

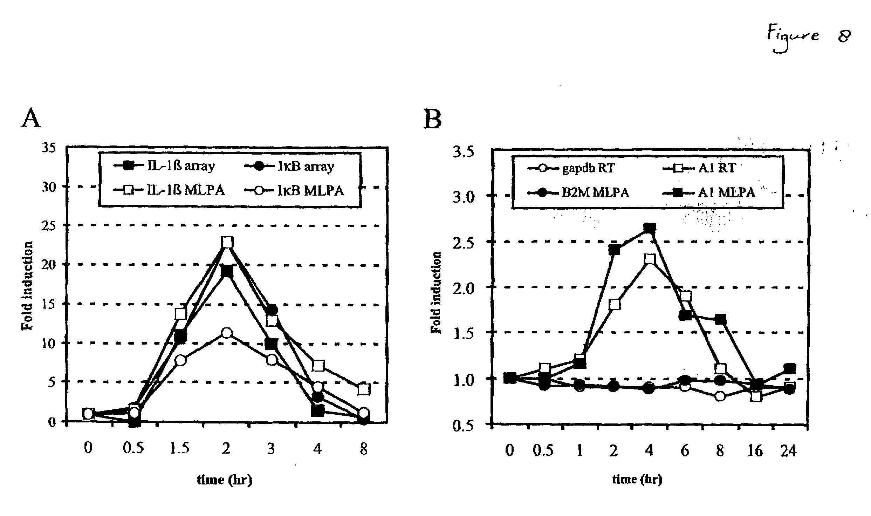

In order to reliably monitor changes in transcription of the target genes, two requirements must be met by a technique containing a PCR stage. First, the signal strength and ratios should not be influenced by the number of PCR cycles. Second, even large changes in expression of a certain gene should not influence the signals of the other probes. These requirements were met, as illustrated by the examples in Figure 6. Varying the number of PCR cycles from 28 to 40 did not significantly influence the signal ratios, both for high and low responses, while the total signal strength increased by a factor of 10. After 28 cycles, very low signals were not yet detectable, but these yielded a constant response from 30 cycles on. Importantly, from 34 to 40 cycles the total signal hardly increased due to exhaustion of reagents (mostly primers), but during these additional cycles the signal ratios remained constant (Fig.6A and B). Next, by titrating in an unlabeled competitor of a high-response gene (BNIP3/NIP3), the relative signal was decreased from 12% to <0.5% of total. The competitor used was identical to the hybridizing part of the short probe oligonucleotide but lacked the PCR primer sequence. This large fluctuation in a single particular gene hardly influenced the other responses, as illustrated in Fig.6C (and by additional data described below). Thus, these results demonstrate that RT-MLPA is well suited to track changes in expression of the target genes. In the MLPA probe mixes used we deliberately reduced the signal of some probes (listed in Table 10 and 11), such as the probe detecting the abundant betamicroglobulin transcript, up to 6-fold by the use of a mixture of short probe oligonucleotides and competitor oligonucleotide.

### Reproducibility and compatibility with other techniques

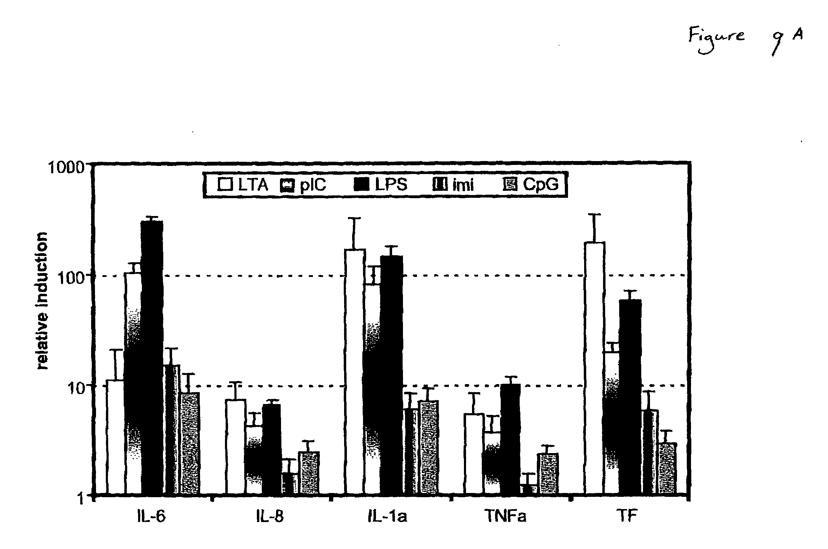

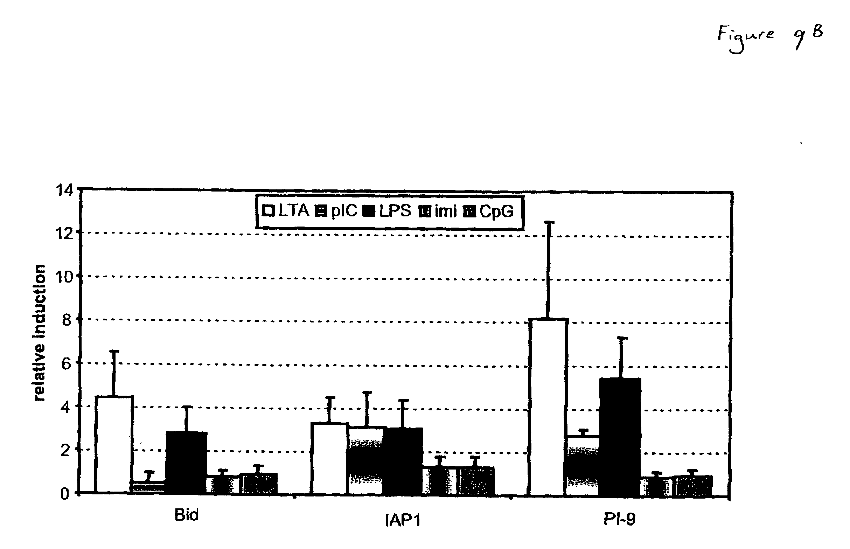

Reliability of results was tested in two ways: by comparing dilutions of identical input samples and by independent duplicate samples. The results in Figure 7 are displayed as relative response of the various target genes related to total signal in the sample (apoptosis probe set), or calculated with reference to the value for the ß2M signal (inflammation probe set). Correlation of the data sets of diluted with undiluted samples was excellent (P>0.99 for 1:10 and 1:100-fold dilution), both for low (<0.5% of total signal) and highly expressed genes. Reproducibility of independent duplicate samples was also satisfactory; interassay correlation between 3 representative data sets of independent samples was 0.96 and ultra-assay variation between 4 independent samples was 0.97. The response of various genes in RT-MLPA was compared to results obtained with cDNA microarrays and standard RT-PCR. Representative examples in Figure 8 illustrate that both high and low variations in response are faithfully detected. Blood samples obtained at timed intervals after administration of LPS to healthy volunteers (13) showed a >10-fold peak response of IL-1β and IkBa at 2 hrs (Fig.8A). Similarly, after antigen receptor triggering of Ramos Burkitt lymphoma cells, expression levels of the protective Bcl-2 family member A1 peaked at 4 hrs with a relative 2.2-2.6- fold increase (Fig.8B), as detected by both RT-PCR and RT-MLPA. For comparison, non-changing expression levels of housekeeping genes are also shown. In summary, RT-MLPA represents a simple and robust technique to monitor (changes in) gene expression.

### Applications

The RT-MLPA probe sets presented here were designed to target two defined biological responses; inflammation and apoptosis. We assumed that specific expression profiles for the targeted genes in separate individuals or distinct cell types could be obtained. In addition, we postulated that the current knowledge with respect to the number of genes involved is sufficient to adequately monitor the most relevant changes in transcription patterns that may govern these processes. A crucial rationale behind the RT-MLPA technique is that it should provide, by relatively simple means, a clear and relevant basis for further experiments relating to the responses under investigation. These assumptions were tested by various approaches.

### Profiles of patients and various cell types

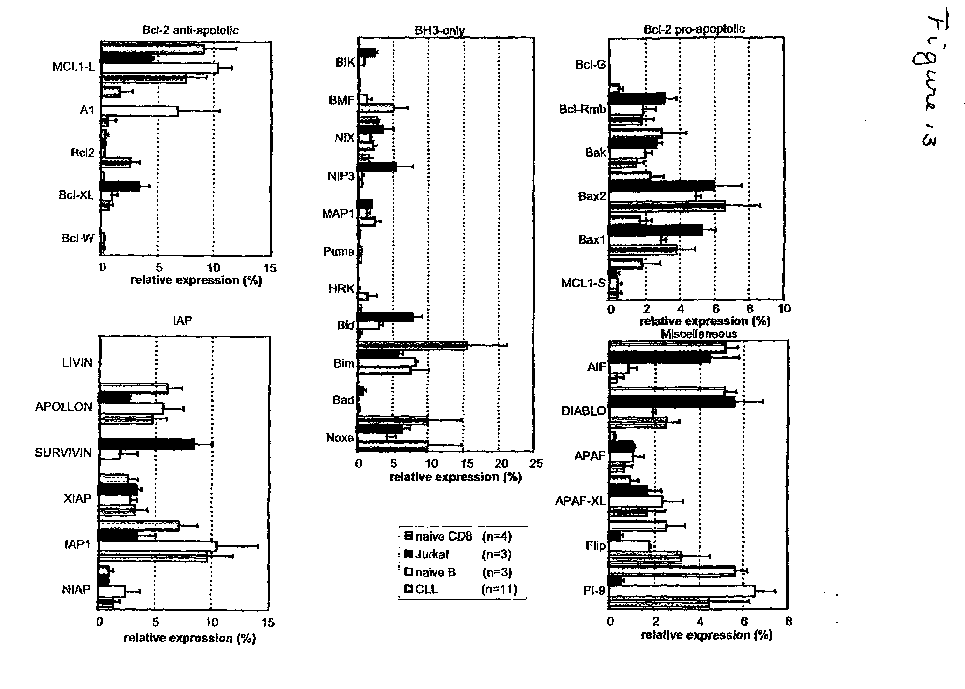

In order to reliably compare (groups of) individuals or patients for response to a stimulus or drug in a desired setting it is important that the expression profiles show equivalent baseline levels of target genes. This was validated for the inflammation probe set in RNA obtained from peripheral blood leucocytes of 16 healthy volunteers before undergoing experimental low grade endotoxemia. The variation in expression levels observed was generally <20% of the total signal for a particular gene, both for low and highly expressed genes (see Fig.12). Conversely, valuable information concerning signaling pathways operating in distinct cell types can be obtained by profiling, especially if the expression patterns are typical for that cell type. To test whether such signature profiles can be obtained by MLPA, we compared naive tonsil B cells and naive CD8 T cells from several donors using the apoptosis probe set. As representatives of malignant B and T cells that may have disturbed responses to apoptotic signals, we included ex vivo samples of chronic lymphocytic leukemia (B-CLL) and Jurkat T cell lines maintained in three separate laboratories. The results are summarized in Fig.13, and clearly show that indeed a particular cell type displays a specific profile. For example, already within the group of proapoptotic BH3-only members of the Bcl-2 family, striking differences were observed, yielding interesting clues about the apoptotic mechanisms that can be active. For more details, see legend to figure 13.

### Induction of inflammation or apoptosis.

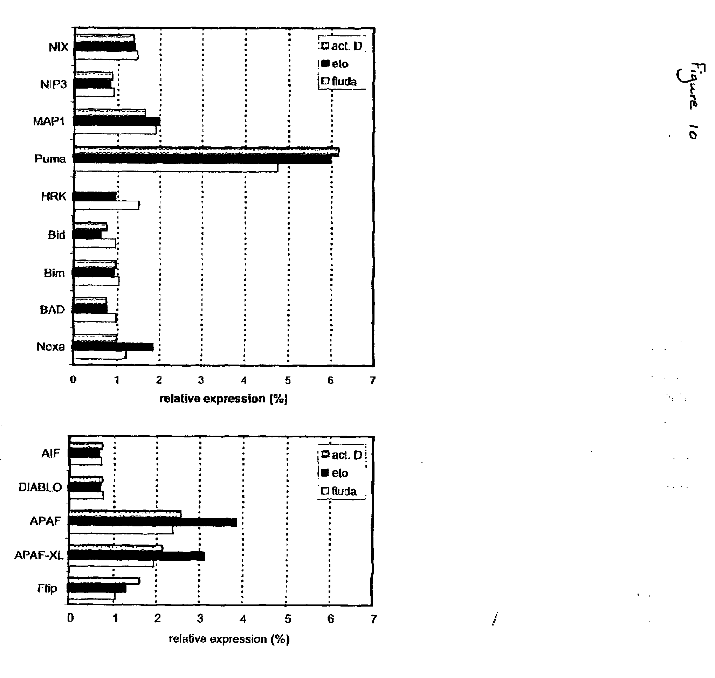

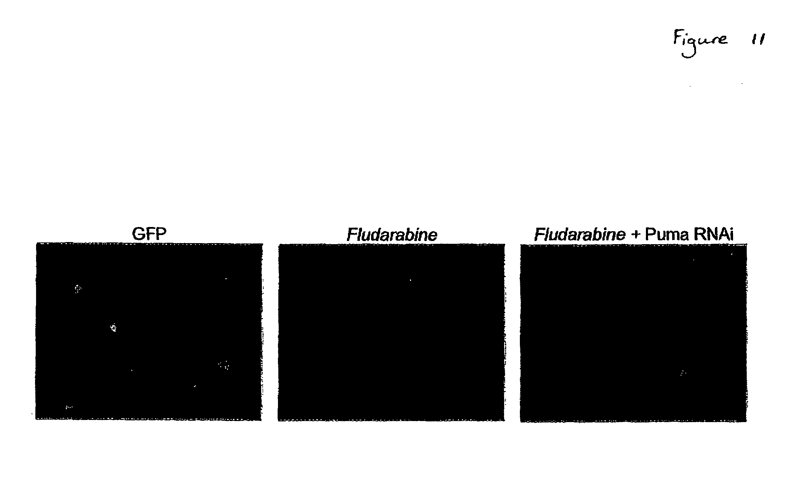

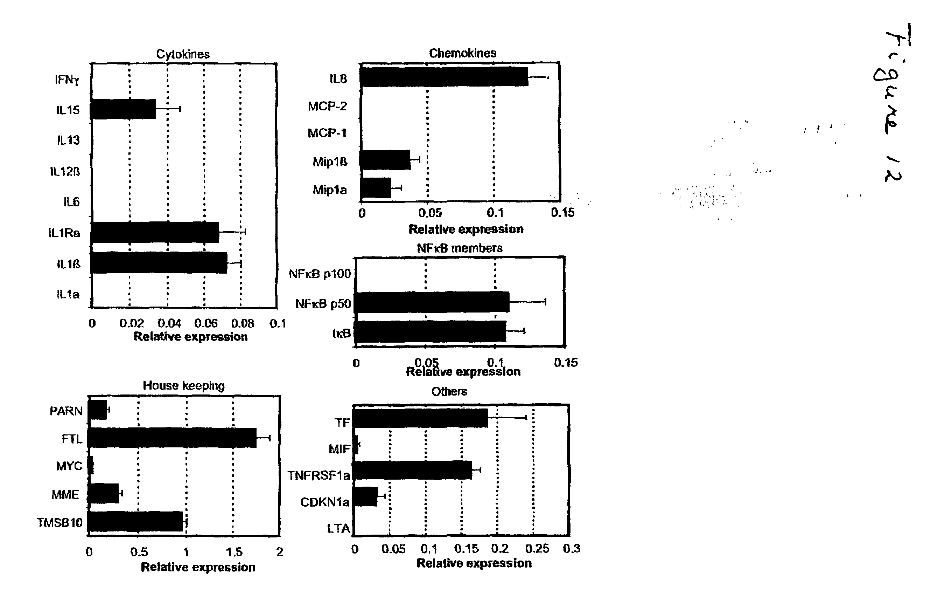

In order to utilize RT-MLPA to accurately determine alterations in gene expression during an inflammatory response, we analysed mRNA expression in unfractionated PBLs stimulated ex vivo with ligands of several innate immunity receptors of the TLR family, known to induce differential gene expression. Incubation of whole blood for 2 hours with LTA, pIC, LPS, imiquimod or CpG , which are ligands for TLR2, 3, 4, 7 and 9, respectively (32), triggered the expression of a restricted set of inflammatory and apoptosis regulatory genes. Induction of expression ranging from 10-200-fold was observed for IL-1a, IL-6 and TF (Fig.9A), as well as MIP-1a and MCP-1(data not shown) after stimulation with ligands for TLR2, 3 and 4, but not TLR7 and 9. The distinct gene expression profiles may well be explained by the differential TLR expression on subsets of PBL and diversity in TLR signaling pathways. The TLRs for LTA and LPS are present on monocytes and PMNs, whereas the TLRs targeted by imiquimod and CpG are present on circulating B cells and plasmacytoid dendritic cells (33-35). Furthermore, TLR2, 7 and 9 signaling induces NF-kB and p38 MAPK activation, whereas TLR4 signaling induces also IFN-ß-inducible genes (32;36). An unexpected observation was that pIC induced a gene profile that closely mimicked the profiles induced by LTA and LPS (including the induction of monocyte-specific Tissue Factor). Although it was previously shown that peripheral blood mononuclear cells are pIC responsive (37), the expression of TLR3 in PBL is still controversial (33;34;38;39). Moreover, the signaling pathway downstream of TLR3 is different from that of TLR2 and 4 (40). Our data, however, implies that pIC acts on unfractionated PBL in a similar manner to LTA or LPS. Besides the induction of inflammatory genes, altered expression of a number of apoptosis regulatory genes was found in PBL after treatment with ligands for TLR2, 3 or 4 (Fig.9B).The most notable effect was found for the granzyme B inhibitor PI-9, which was strongly upregulated by LTA, pIC and LPS, confirming and extending recent findings that PI-9 is induced in liver cells by LPS under control of NF.B and AP-1 (41). Other genes induced by the same stimuli were Bid and IAP1, but in contrast transcripts for Puma and Apaf were suppressed approximately 5-fold (not shown). Taken together, these data indicate that the RT-MLPA method efficiently detects changes in expression of major effectors during inflammatory processes, and is well suited to monitor differential patterns of gene induction and repression. To monitor gene induction during apoptosis, we utilized U2OS osteosarcoma cells treated with various cytotoxic agents. These cells are a well-known model system and have an intact p53 response (42;43). After treatment with the DNA-modifying prodrug fludarabine, the topoisomerase inhibitor etoposide or RNA-synthesis inhibitor actinomycin D, expression of apoptotic regulators was determined via RT-MLPA. The results in Fig. 10 show a distinct and prominent induction of the transcript encoding the BH3-only protein Puma. This apoptosis inducer was characterized recently based on its early upregulation upon p53 activation (42;44), and was shown to trigger cytochrome C release form mitochondria. The only other gene clearly upregulated was the co-factor for apoptosome formation Apaf (45), which is also known to be p53-responsive (46). Additional genes reported to be p53-reactive such as Bax and Noxa (47) were induced to a much lower extent, in good agreement with a very recent report on the -p53 response in colorectal cancer cells (48). Certain genes were clearly downregulated in response to all three agents, such as the IAP family members Survivin and Apollon (data not shown). The obvious and testable conclusion from this experiment is that after cytostatic drug treatment in these cells, upregulation via p53 of PUMA is the prime inducer of apoptosis. This was verified by an RNAi approach. U2OS were transfected with empty pSuper vector (49) or 2 constructs targeting the Puma transcript, and subsequently treated with fludarabine for 24 hrs (Fig. 11). The panels show respectively healthy adherent cells transfected with GFP plasmid only (left), non-adherent dead cells in the presence of fludarabine (middle), and again predominantly healthy cells by combining Puma RNAi with drug treatment. Clearly, suppression of Puma expression resulted in increased survival of cells in the presence of the drug. This approach thus quickly yields insight into relevant responses of malignant cells to cytostatic drugs.

### DISCUSSION

We describe and validate a novel technique to perform easy and reliable expression profiling of genes. Relevant examples presented here concern two crucial biological processes; inflammation and apoptosis. RT-MLPA has important advantages over current techniques such as Northern blotting, real-time PCR, RNase protection assays and even large scale micro-array approaches. Numerous samples can be quickly processed in a standard PCR 96-well format, which can be easily adapted for automation. Analysis is straightforward and yields quantitative information on a medium-sized gene number. These are obvious improvements compared to Northern blotting, real-time PCR and RNase protection assays, which are either quite laborious, less sensitive and/or more limited in scope. In addition, these prior art techniques may require cumbersome radioactive techniques for maximum sensitivity.

Because a predefined set of genes is targeted by MLPA instead of the whole genome, a trade-off is made between scope of the analysis and ease of interpretation. Although micro array techniques potentially sample the whole transcriptome, in practice functional interpretation is limited when hundreds of genes are differentially expressed, which is often the case; see e.g. (50, 51). In many instances, lists of gene products identified by microarray cover every aspect of cellular biochemistry and function. In addition, the time consuming design and fabrication of large scale solid phase micro-arrays may preclude incorporation of novel genes, while with RT-MLPA new probes can be quickly included in an existing probemix should this need arise. To illustrate this point, of the apoptosis regulators represented in the current probe mix, a significant number (9-11, i.e. 28-34%) is lacking on two microarrays we have investigated (Netherlands Cancer Institute www.microarrays.nki.nl, and lymphochip www.llmpp.nih.gov). Most prominent among these was PUMA, which we could pinpoint with RT-MLPA as the prime apoptosis inducer in a model of cytostatic drug treatment. In addition, both microarrays lacked representation of Bcl-rambo, Bcl-GS, Apollon, Livin, Map-1 and Smac-Diablo, and various others were absent from a single array. Curiously, omissions were not limited to novel or obscure genes, suggesting completeness within a specified area is difficult to achieve with unbiased large scale set ups.

Using RT-MLPA, only 5 ng of total input RNA can yield reliable quantitative information. This makes possible direct analysis of minute samples obtained from, for example, cell sorting or needle biopsies, without the need for prior amplification. Although amplification strategies improve RNA yield to levels that allow labeling and detection by microarray, this still requires substantially more (³50-fold) starting material than RT-MLPA. Furthermore, although amplification may preserve general overall representation, a certain distortion is inevitable and especially low abundance transcripts may be lost (52). Also, solid phase hybridisation techniques with cDNA microarrays often cannot discriminate highly homologous genes, while with MLPA such genes can be targeted by specifically designed probes. Thus, for various current purposes, the sensititivity and the focus on an entire set of genes provided by MLPA is in fact superior to microarray techniques.

The RT-MLPA procedure proved sensitive, consistent and highly concordant with RT-PCR and microarray results over a wide range of input RNA concentrations. The highest induction observed in the experiments described here was over 100-fold. By including non-amplifiable competitor-oligo's in an MLPA probe-mix, high signals can be attenuated to a desired level, as exemplified here in Figure 6C.

In conclusion, RT-MLPA provides a valuable contribution to expression profiling. The technique is well suited for high-throughput automated applications, such as for instance delineation of gene expression in large case-control studies of anti-inflammatory drugs. Another promising current approach includes the mechanism of apoptotic drugs in various malignancies, and to assemble potentially discriminating profiles in responders versus non-responders to therapy.

**Table 1:**

| Alphabetical listing of the mRNAs and median values of cases and control subjects and their range. | | | | | | |
|---|---|---|---|---|---|---|
| Gene symbol | Descriptive name | Median Cases | Range | Median Controls | Range | ^{¶}p-value |
| B2M | Beta-2-microglobulin | * | * | * | * | |
| BM1I | BMI-1 oncogene homolog | 0.84 | 0.09-2.18 | 0.76 | 0.00-2.41 | <0.001 |
| CCL3 | Chemokine (C-C motif) ligand 3 | 0.14 | 0.00-0.85 | 0.13 | 0.00-1.16 | <0.001. |
| CCL4 | Chemokine (C-C motif) ligand 4 | 1.30 | 0.23-* | 1.26 | 0.23-* | <0.001 |
| CDKN1A | Cyclin-dependent kinase inhibitor 1A | - | - | - | - | NA |
| GSTP1 | Glutathione S-transferase | 0.31 | 0.00-1.36 | 0.29 | 0.00-1.05 | <0.000 |
| IFNG | Interferon, gamma | 0.16 | 0.00-0.80 | 0.15 | 0.00-1.78 | <0.207 |
| IL-10 | Interleukin 10 | 0.00 | 0.00-0.73 | 0.00 | 0.00-0.45 | <0.006 |
| IL12A | Interleukin 12, subunit p35 | 3.04 | 0.16-* | 2.76 | 0.16-* | <0.001 |
| IL12B | Interleukin 12, subunit p40 | 0.00 | 0.00-0.33 | 0.00 | 0.00-1.78 | <0.06 |
| IL13 | Interleukin 13 | 0.00 | 0.00-0.26 | 0.00 | 0.0-0.08 | <1.0 |
| IL15 (1) | Interleukin 15, transcript variants I and 3 | 0.48 | 0.13-2.59 | 0.46 | 0.16-2.52 | <0.015 |
| IL15 (2) | Interleukin 15, transcript variant 2 | 0.00 | 0.00-0.26 | 0.00 | 0.00-0.08 | <0.003 |
| IL18 | Interleukin 18 | 0.12 | 0.00-1.45 | 0.11 | 0.00-0.97 | <0.01 |
| IL1A | Interleukin 1, alpha | 0.00 | 0.00-0.39 | 0.00 | 0.00-0.31 | <0.001 |
| IL1B | Interleukin 1, beta | 0.99 | 0.24-* | 0.87 | 0.14-* | <0.001 |
| IL-1RN | Interleukin 1 receptor antagonist | 1.90 | 0,54-* | 1.71 | U.47-* | <0.001 |
| IL2 | Interleukin 2 | 0.07 | 0.00-0.52 | 0.03 | 0.00-1.19 | <0.001 |
| IL4 (1) | Interleukin 4, transcript variant 1 | 0.00 | 0.00-0.31 | 0.00 | 0.00-0.22 | <0.8 |
| IL4 (2) | Interleukin 4, transcript variants 1 and 2 | 0.03 | 0.00-0.46 | 0.00 | 0.00-0.51 | <0.001 |
| IL6 | Interleukin 6 | 0.00 | 0.00-0.24 | 0.00 | 0.00-0.28 | <0.2 |
| IL8 | Interleukin 8 | 1.38 | 0.00-* | 1.30 | 0.00-* | <0.001 |
| LTA | Lymphotoxin alpha (Tumor necrosis factor, beta) | 0.25 | 0.00-1.08 | 0.23 | 0.00-0.82 | <0.001 |
| MCP-1 | Monocyte chemotactic protein, 1 | 0.00 | 0.00-8.22 | 0.14 | 0.00.0.70 | <0.4 |
| MCP-2 | Monocyte chemotactic protein, 2 | 0.00 | 0.00-10.36 | 0.00 | 0.00-0.66 | <0.08 |
| MIF | Macrophage migration inhibitory factor | 0.91 | 0.00-3.95 | 0.75 | 0.00-3.98 | <0.001 |
| MYC | v-myc oncogene homolog | 1.41 | 0.43-* | 1.25 | 0.28-* | <0.001 |
| NFKB 1 | nuclear factor kappa-B, subunit 1 | 1.12 | 0.43-* | 1.02 | 0.19-* | <0.001 |
| NFKB2 | nuclear factor kappa-B, subunit 2 | 0.00 | 0.00-0,29 | 0.00 | 0.00-0.20 | <0.05 |
| NFKBIA | nuclear factor kappa-B, inhibitor. alpha | 2.98 | U.65-* | 2.66 | 0.55-* | <0.001 |
| PARN | Polyadenylate-specific ribonuclease | 2.27 | 0.71-* | 2.34 | 0.67-* | <0.5 |
| PDE4B | Phosphodiesterase 4B, CAMP-specific | 2.23 | 0.54-* | 2.12 | 0.58-* | <0.001 |
| PDGFB | Platelel-derived growth factor, beta polypeptide | 0.15 | 0.00-0.65 | 0.13 | 0.00-0.75 | <0.002 |
| PI9 | proteinase inhibitor 9, ovalbumin type | 1.81 | 0.24-* | 1.58 | 0.00-* | <0.001 |
| PTP4A2 | Protein-tyrosine phosphatase, type 4A, 2 | * | * | * | * | |
| PTPN1 | Protein-tyrosine phosphatase, nonreceptor-type, 1 | 0.24 | 0.00-0.63 | 0.21 | 4.00-0.79 | <0.001 |
| TF | Tissue factor | 0.00 | 0.00-0.13 | 0.00 | 0.00-0.07 | <0.6 |
| THBS1 | Thrombospondin 1 | 0.29 | 0.00-1.47 | 0.30 | 0.00-1.40 | <0.8 |
| TNF | Tumor necrosis factor, alpha | 0.00 | 0.00-0.32 | 0.00 | 0.00-0.21 | <0.015 |
| TNFRSF1A | Tumor necrosis factor reccptor superfamily, 1A | 2.77 | 1.02-* | 2.57 | 0.77-* | <0.001 |

| | | | | | | |
|---|---|---|---|---|---|---|
| *mRNA levels ahove the detection limit; | | | | | | |
| ^{¶}according to the (2-tailed) Mann-Whitney test. | | | | | | |

**Table 2,**

| Characteristics of patients and control subjects. | | |
|---|---|---|
| | Cases (524) | Controls (628) |
| Age, years (mean) | 56.4 | 57.4 |
| Current smokers (%) | 323 (61.6) | 209 (33.3) |
| Alcohol users (%) | 420 (80.2) | 543 (86.5) |
| *Obesity (%) | 90 (17.2) | 104 (16.6) |
| Diabetes (%) | 23 (4.4) | 21 (3.3) |
| ^{§}Hypertension (%) | 97 (18.5) | 101 (16.1) |
| ^{§}(iypercholesterolemia (%) | 12 (2.3) | 10(1.6) |

| | | |
|---|---|---|
| *A person was defined as obese if his BMI exceeded 30 kg/m². Data on height and weight were not available for two people. | | |
| ^{§} A person was classified as hypertensive or hypcreholesterolemic if he was taking prescription drugs for these conditions. | | |

**Table 3.**

| Odds Ratios for highest versus lowest quartile. Results of a stratified analysis on men who were non-smokers both at the index date and the sampling date are shown in the last column after adjustment for age. 95% Confidence Interval is shown in brackets. | | | | | |
|---|---|---|---|---|---|
| Inflammatory Marker | Cases (N=524) | Controls (N=628) | Crude OR | Adjusted OR* | OR for non-smokers only, age corrected (N=617) |
| MIF | 193 | 157 | 3.4 (2.3-4.9) | 3.0 (2.0-4.4) | 5.2 (2.9-9.5) |
| PI9 | 195 | 156 | 2.5 (1.8-3.5) | 2.6 (1.8-3.8) | 3.1 (1.8-5.3) |
| CCL3 | 183 | 156 | 2.2 (1.5-3.0) | 2.3 (1.6-3.3) | 2.8 (1.7-4.6) |
| PTPN1 | 170 | 156 | 2.4 (1.7-3.4) | 2.2(1.5-3.2) | 3.7 (2,1-6.3) |
| BMI1 | 158 | 157 | 1.9 (1.3-2.6) | 2.0 (1.4-2.9) | 1.7 (1.0-2.8) |
| PDE4B | 162 | 157 | 1.9 (1.3-2.6) | 1.9 (1.3-2.8) | 2.7 (1.6-4.6) |
| GSTP1 | 162 | 148 | 1.9 (1.3-2.6) | 1.8 (1.2-2.5) | 1.7 (1.0-2.8) |
| NFKB1 | 182 | 157 | 2.0 (1-4-2.8) | 1.7 (1.2-2.4) | 2.0 (1.2-3.3) |
| IL12A | 165 | 157 | 1.8 (1.3-2.5) | 1.7 (1.2-2.4) | 2.2 (1.3-3.6) |
| PDGFB | 156 | 156 | 1.6 (1.1-2.2) | 1.7(1.2-2.4) | 1.3(0.8-2.2) |
| MYC | 182 | 157 | 2.0 (1.4-2.8) | 1.6 (1.1-2.3) | 1.6 (1.0·2.6) |
| IL1B | 193 | 157 | 2.0 (1.5-2.9) | 1.6 (1.1-2.3) | 2.7(1.6-4.5) |
| IL8 | 152 | 157 | 1.6 (1.1-2.2) | 1.6 (1.1-2.3) | 2.0 (1.2-3.3) |
| NFKBIA | 173 | 157 | 1.7 (1.2-2.3) | 1.5 (1.0-2.1) | 1.9 (1.2-3.1) |
| IL15(1) | 139 | 157 | 1.4 (1.0-2.0) | 1.5 (1.0-2.1) | 1.5 (0.9-2.6) |
| LTA | 163 | 156 | 1.7 (1.2-2.4) | 1.4 (1.0-2.0) | 1.4 (0.8-2.3) |
| IFNG | 146 | 157 | 1.3 (0.9-1.8) | 1.4 (1.0-2.1) | 1.5 (0.9-2.4) |
| IL1RN | 160 | 157 | 1.7 (1.2-2.3) | 1.3 (0.9-1.9) | 1.4 (0.8-2.3) |
| TNFRSFIA | 153 | 157 | 1.4 (1.0-2.0) | 1.3 (0.9-1.8) | 1.9 (1.2-3.1) |
| IL18 | 132 | 156 | 1.3 (0.9-1.8) | 1.3(0.9-1.8) | 1.5 (0.9-2.5) |
| CCL4 | 135 | 157 | 1.0 (0.7-1.4) | 1.3 (0.9-1.9) | 0.9 (0.6-1.5) |
| THB51 | 126 | 156 | 0.9 (0.6-1.2) | 0.9 (0.7-1.3) | 0.7 (0.5-1.2) |
| PARN | 117 | 157 | 0.8 (0.6-1.2) | 1.0 (0.7-1,5) | 0.7 (0.4-1.1) |

| | | | | | |
|---|---|---|---|---|---|
| *Adjusted for age, smoking, hypertension, hypercholesterolemia, diabetes, BMI, alcohol habit and quartile of CRP. | | | | | |

**Table 4.**

| Odds Ratios with increasing quartiles of mRNA levels for a selection of markers. 95% Confidence Interval is shown in brackets. The 1st quartile is always the reference category. | | | | | |
|---|---|---|---|---|---|
| Inflammatory Marker | Quartile | Cases (N=524) | Controls (N=628) | Crude OR | Adjusted OR* |
| MIF | 1st quartile | 57 | 157 | 1 | 1 |
| | 2nd quartile | 131 | 157 | 2.3 (1.6-3.4) | 2.1 (1.4-3.2) |
| | 3rd quartile | 143 | 157 | 2.5 (1.7-3.7) | 2.4 (1.6-3.6) |
| | 4th quartile | 193 | 157 | 3.4 (2.3-4.9) | 3.0 (2.0-4.4) |
| PI9 | 1 | 78 | 157 | 1 | 1 |
| | 2 | 106 | 157 | 1.4 (1.0-2.0) | 1.4 (1.0-2.1) |
| | 3 | 145 | 157 | 1.9 (1.3-2.6) | 1.8 (1.2-2.6) |
| | 4 | 195 | 156 | 2.5 (1.8-3.5) | 2.6 (1.8-3.8) |
| PTPN1 | 1 | 73 | 158 | 1 | 1 |
| | 2 | 122 | 155 | 1.7 (1.2-2.5) | 1.7 (1.2-2.5) |
| | 3 | 159 | 155 | 2.2 (1.6-3.2) | 2.0 (1.4-2.9) |
| | 4 | 170 | 156 | 2.4 (1.7-3.4) | 2.2 (1.5-3.2) |
| PDGFB | 1 | 98 | 157 | 1 | 1 |
| | 2 | 124 | 156 | 1.3 (0.9-1.8) | 1.3 (0.9-1.9) |
| | 3 | 146 | 156 | 1.5 (1.1-2.1) | 1.5 (1.0-2.2) |
| | 4 | 156 | 156 | 1.6 (1.1-2.2) | 1.7 (1.2-2.4) |
| IL12A | 1 | 92 | 157 | 1 | 1 |
| | 2 | 126 | 157 | 1.4(1.0-1.9) | 1.3(0.9-1.9) |
| | 3 | 141 | 157 | 1.5 (1.1-2.2) | 1.4 (1.0-2.1) |
| | 4 | 165 | 157 | 1.8 (1.3-2.5) | 1.7 (1.2-2.4) |
| NFKB1 | 1 | 90 | 157 | 1 | 1 |
| | 2 | 108 | 157 | 1.2 (0.8-1.7) | 1.2(0.8-1.7) |
| | 3 | 144 | 157 | 1.6(1.1-2.3) | 1.4(1.0-2.1) |
| | 4 | 182 | 157 | 2.0 (1.4-2.8) | 1.7 (1.2-2.4) |
| MYC | 1 | 91 | 157 | 1 | 1 |
| | 2 | 96 | 157 | 1.0 (0.7-1.5) | 1.0 (0.7-1.4) |
| | 3 | 155 | 157 | 1.7 (1.2-2.4) | 1.5 (1.0-2.1) |
| | 4 | 182 | 157 | 2.0 (1.4-2.8) | 1.6 (1.1-2.3) |
| LTA | 1 | 97 | 157 | 1 | 1 |
| | 2 | 107 | 157 | 1.1 (0.8-1.6) | 1.0 (0.7-1.5) |
| | 3 | 157 | 156 | 1.6 (1.2-2.3) | 1.3 (0.9-1.9) |
| | 4 | 163 | 156 | 1.7 (1.2-2.4) | 1.4 (1.0-2.0) |

**Table 5.**

| Odds Ratios for markers with readings above or below the detection limits. Results of a stratified analysis on men who were non-smokers at the index date and sampling date are shown in the last column. 95% Confidence Interval is shown in brackets. | | | | | |
|---|---|---|---|---|---|
| Inflammatory Marker | Cases (N=524) | Controls (N=628) | Crude OR | Adjusted OR* | OR for non-smokers only, age corrected (N=617) |
| Detectable versus non-detectable levels of relative mRNA | | | | | |
| IL15(2) | 42 | 25 | 2.1 (1.3-3.5) | 2.2 (1.3-3.7) | 2.7 (1.4-5.5) |
| MCP-2 | 31 | 23 | 1.7 (1.0-2.9) | 1.8 (1.0-3.2) | 2.1 (1.0-4.5) |
| IL2 | 334 | 316 | 1.7 (1.4-2.2) | 1.7 (1.3-2.2) | 2.0 (1-4-2.8) |
| IL12B | 57 | 48 | 1.5 (1.0-2.2) | 1.6 (1.1-2.5) | 1.5(0.8-2.6) |
| IL6 | 49 | 43 | 1.4 (0.9-2.1) | 1.4 (0.9-2.3) | 1.4 (0.7-2.6) |
| TNF | 76 | 62 | 1.5 (1.1-2.2) | 1.4(1.0-2.1) | 1.7 (1.0-2.9) |
| IL4(2) | 266 | 248 | 1.6 (1.3-2.0) | 1.4(1.1-1.8) | 1.7 (1.2-2.4) |
| IL10 | 58 | 42 | 1.7(1.1-2.6) | 1.4 (0.9-2.1) | 2.3 (1.2-4.4) |
| IL13 | 4 | 5 | 1.0 (0.3-3.6) | 1.2 (0.3-5.0) | 0.5 (0.1-4.9) |
| TF | 8 | 7 | 1.4 (0.5-3.8) | 1.2 (0.4-3.7) | 0.4 (0.0-3.7) |
| MCP-1 | 120 | 128 | 1.2 (0.9-1.5) | 1.2 (0.9-1.6) | 1.0 (0.6-1.5) |
| NFK82 | 152 | 152 | 1.3 (1.0-1.7) | 1.2 (0.9-1.6) | 1.4 (0.9-2.0) |
| IL4(1) | 110 | 138 | 1.0 (0.7-1.3) | 0.9 (0.7-1.2) | 0.9 (0.6-1.4) |
| IL1A | 189 | 291 | 0.7 (0.5-0.8) | 0.6 (0.5-0.8) | 0.5 (0.4-0.7) |
| Marker with levels above the upper detection limit versus levels within the detection limits | | | | | |
| PTP4A2** | **428 | **437 | 1.8 (1.3-2.3) | 1.7 1.2-2.3) | #1.8 (1.2-2.7) |

| | | | | | |
|---|---|---|---|---|---|
| *Adjusted for age, smoking, hypertension, hypercholesterolemia, diabetes, BMI, alcohol habit and quartile of CRP. | | | | | |
| **Out of 518 cases and 599 controls. | | | | | |
| # Out of 592 samples | | | | | |

**Table 6:**

| **Inflammatory genes as analysed by MLPA.** | | |
|---|---|---|
| **Functional category** | **Gene** | **Symbol** |
| Cytokines | Interleukin-1 alpha | 1L-1α |
| | Interlcukin-1 beta | I1-1β |
| | Interleukin-1 receptor antagonist | IL-1Ra |
| | Interleukin-6 | lL-6 |
| | Interleukin-12 (p40) | IL-12 β |
| | Interleukin-13 | IL-13 |
| | Interleukin-15 | IL-15 |
| | Interferon gamma | IFNγ |
| Chemokines | Interleukin-8 | IL-8 |
| | Monocyte chemotactic protein I | MCP-1 |
| | Monocyte chemotactic protein 2 | MCP-2 |
| | Macrophage inflammatory protein 1 alpha | MTP-1α |
| | Macrophage inflammatory protein 1 beta | MIP-1β |
| NTκB members | Nuclear factor kappa beta inhibitor alpha | IκBα |
| | Nuclear factor kappa beta p50 | p50 |
| | Nuclear factor kappa beta p49/p100 | p49/p100 |
| Coagulation factor | Tissue factor | TF |
| Control gene | Beta-2-microglobulin | B2M |
| | Ferritin light chain | FTI. |
| Others | Tumor necrosis factor receptor super family member 1 | TNFR 1 |

**Table 7**

| Leukocyte counts and differentials before and after LPS injection, and the influence of IC14 treatment hereon. | | | | | | |
|---|---|---|---|---|---|---|
| **Time (hours)** | **WBCs (x 10**^{**9**}**/L)** | | **Neutrophils (x 10**^{**9**}**/L)** | | **Monocytes (x 10**^{**9**}**/L)** | |
| | **LPS** | **IC14** | **LPS** | **IC14** | **LPS** | **IC14** |
| 0 | 6.8 ± 0.45 | 7.5 ± 0,90 | 3.89 ± 0.44 | 4.96 ± 0.89 | 0.52 ± 0.05 | 0.36 ± 0.05 |
| 0.5 | 6.6 ± 0.42 | 7.8 ± 1.00 | 3.92 ± 0.38 | 5.05 ± 0.85 | 0.40 ± 0.04 | 0.34 ± 0.06 |
| 1 | 2.7 ± 0.38 | 7.4 ± 0.90 | 1.35 ± 0.32 | 4.16 ± 0.43 | 0.05 ± 0.01 | 0.25 ± 0.05 |
| 2 | 7.1 ± 1.10 | 5.7 ± 1.20 | 6.04 ± 1.10 | 2.68 ± 0.39 | 0.05 ± 0.01 | 0.09 ± 0.04 |
| 3 | 7.2 ± 0.80 | 10.4 ± 0.80 | 6.68 ± 0.76 | 7.62 ± 1.20 | 0.04 ± 0.01 | 0.06 ± 0.02 |
| 4 | 11.7 ± 1.00 | 10.3 ± 1.10 | 11.12 ± 0.94 | 9.05 ± 0.96 | 0.12 ± 0.05 | 0.19 ± 0.05 |
| 6 | 15.8 ± 1.10 | 11.1 ± 0.70 | 14,59 ± 1.20 | 9.79 ± 0.69 | 0.36 ± 0.07 | 0.45 ± 0.07 |
| 8 | 17.9 ± 1.50 | 10.7 ± 0.70 | 17.41 ± 1.20 | 9.65 ± 0.68 | 0.51 ± 0.08 | 0.52 ± 0.07 |
| 21 | 12.9 ± 1.40 | 7.5 ± 0.60 | 9.39 ± 1.30 | 4.25 ± 0.43 | 0.85 ± 0.04 | 0.79 ± 0.35 |
| Data are means ± SEM of eight healthy subjects injected i.v. with LPS and placebo or LPS and IC14. Especially note the difference in cell counts 1 hour after LPS injection. | | | | | | |

**Table 8**

| The effect of CD14 on LPS induced mRNA and protein levels in human volunteers during low-grade endotoxemia. All genes for which protein fevels are available are shown. | | |
|---|---|---|
| **Gene** | **Effect CD14 on protein level** | **Effect CD14 on mRNA level** |
| TNFα | Complete inhibition | ND |
| IL-6 | Complete inhibition | ND |
| IL-8 | Complete inhibition | 2 fold inhibition, 1 hour delay |
| sTNFR1 | No inhibition, 1 hour delay | LPS unresponsive, no effect IC14 |
| IL-1Ra | No inhibition, 1 hour delay | 4 fold inhibition, 1 hour delay |
| MIP-1α | No inhibition, no delay | 2 fold inhibition, 1 hour delay |
| MfP-1β | 3 fold inhibition, no delay | 3 fold inhibition, 1 hour delay |
| MCP-1 | 3 fold inhibition, no delay | ND |
| ND: mRNA expression level below the detection limit during the whole time frame of the experiment. The protein data are derived from earlier publications [13,18]. | | |

**Table 9**

| **PROBE SETS** | | | | | | | |
|---|---|---|---|---|---|---|---|
| **A: Inflammatory and NFκB gene set.** | | | | | | | |
| **Cytokines** | **Chemokines** | **NFκB members** | | **Miscellaneous** | | **House keeping** | |
| IL-1α | IL-8. | IκBa | | TF | | B2M | |
| IL-1β | MCP-1 | p50 | | TNFR1 | | FTL | |
| IL-1Ra | MCP-2 | p49/p100 | | MIF | | PARN | |
| IL-6 | MIP-1α | p65 | | CDKN1α | | MYC | |
| IL-10 | MIP-1β | | | LTa | | MME | |
| IL-12β | | | | | | TMSB10 | |
| IL-13 | | | | | | | |
| IL-15 | | | | | | | |
| IFNγ | | | | | | | |
| TNF-α | | | | | | | |

| **B: Apoptosis gene set** | | | | | | | |
|---|---|---|---|---|---|---|---|
| **BCI-2-family** | | | **IAP family** | | **Miscellaneous** | | |
| **Bcl2-like. anti-apoptotic** | **Box-like pro-apoprotic** | **BH3-only pro-apoptotic** | **anti-apoptotic** | | **pro-apoptotic** | | **anti-apoptotic** |
| A1/Bfl-1 | BAK | Bad | Apollon | | AIF | | Flip |
| Bel-W | Bax (2variants) | Bid | IAP1 | | Apaf-1 | | PI-9 |
| Bcl-X1 | Hcl-GS | Bik | IAP2 | | Apaf-IL | | Apaf-IXL |
| Bcl-2 | Bcl-Rambo | Bim | Livin | | Smac/DIABLO | | |
| Mcll-long | Mcll-short | Bmf | NIAP | | | | |
| | | BNIP3/NIP3 | Survivin | | | | |
| | | BNIP3L/Nix | XIAP | | | | |
| | | Harakiri | | | | | |
| | | MAP-1 | | | | | |
| | | Noxa | | | | | |
| | | Puma | | | | | |

**TABLE 1:** Genes targeted by MLPA assay in the inflammation and apoptosis probe sets. Detailed information regarding probe sequence and location, gene name and amplification product length can be found in the supplementary information.
A: Genes and categories for the inflammation probe set. Probes for TNFα, IL-10 and p65 did not yield a signal in samples obtained from peripheral blood, and are therefore not represented in the figures. The various housekeeping genes included in the probe mix are indicated in the right column.
B: Genes and categories for the apoptosis probe set. Pro-apoptotic Bax is targeted by probes discriminating two splice variants (see www.ncbi.nlm.nih.gov/LocusLink). The transcript encoding Apaf-XL was proposed to have anti-apoptotic function (35) and is for that reason tentatively presented as such. Various probes were designed and tested for IAP2, but for unknown reasons these never gave a significant signal. Signals for Bcl-w, Bcl-GS, Livin (also called ML-IAP) were generally very low and/or rare, in accord with their restricted expression. Housekeeping genes included in the apoptosis probe set are β2M, FTL and PARN.

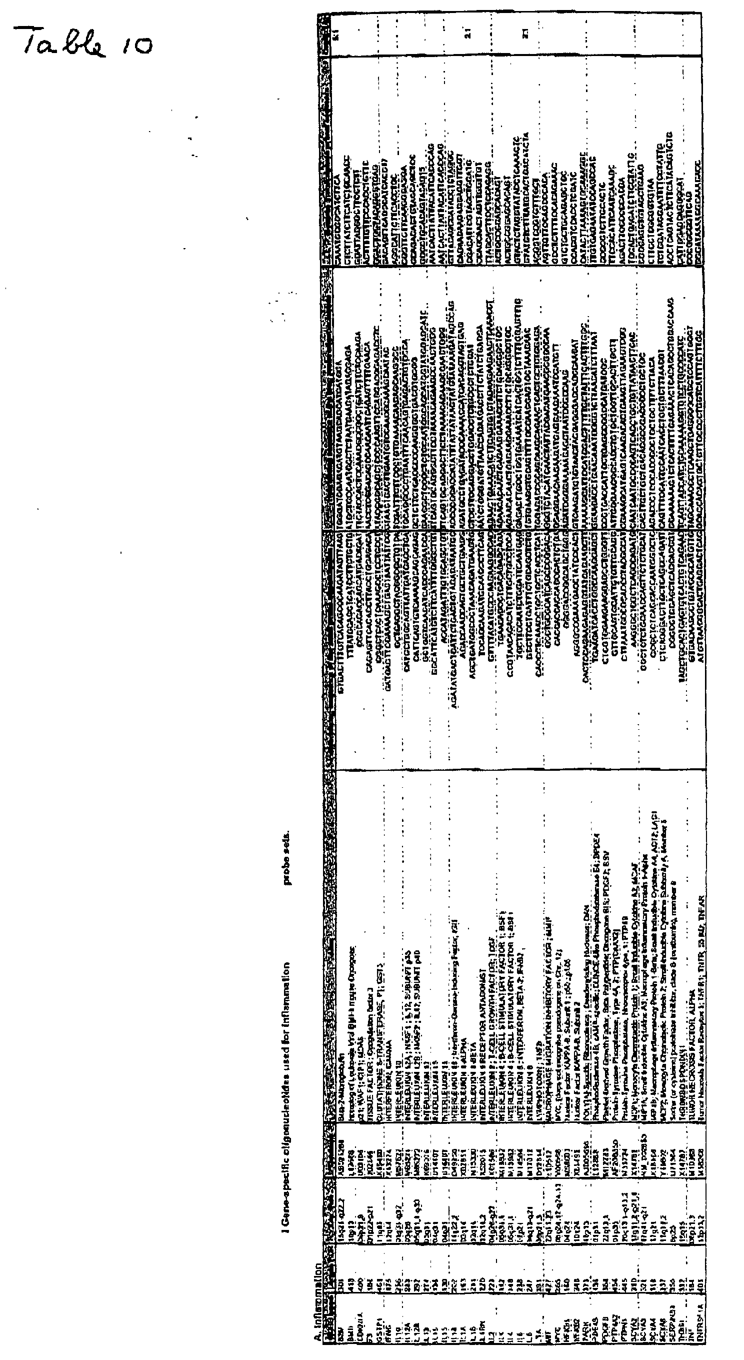

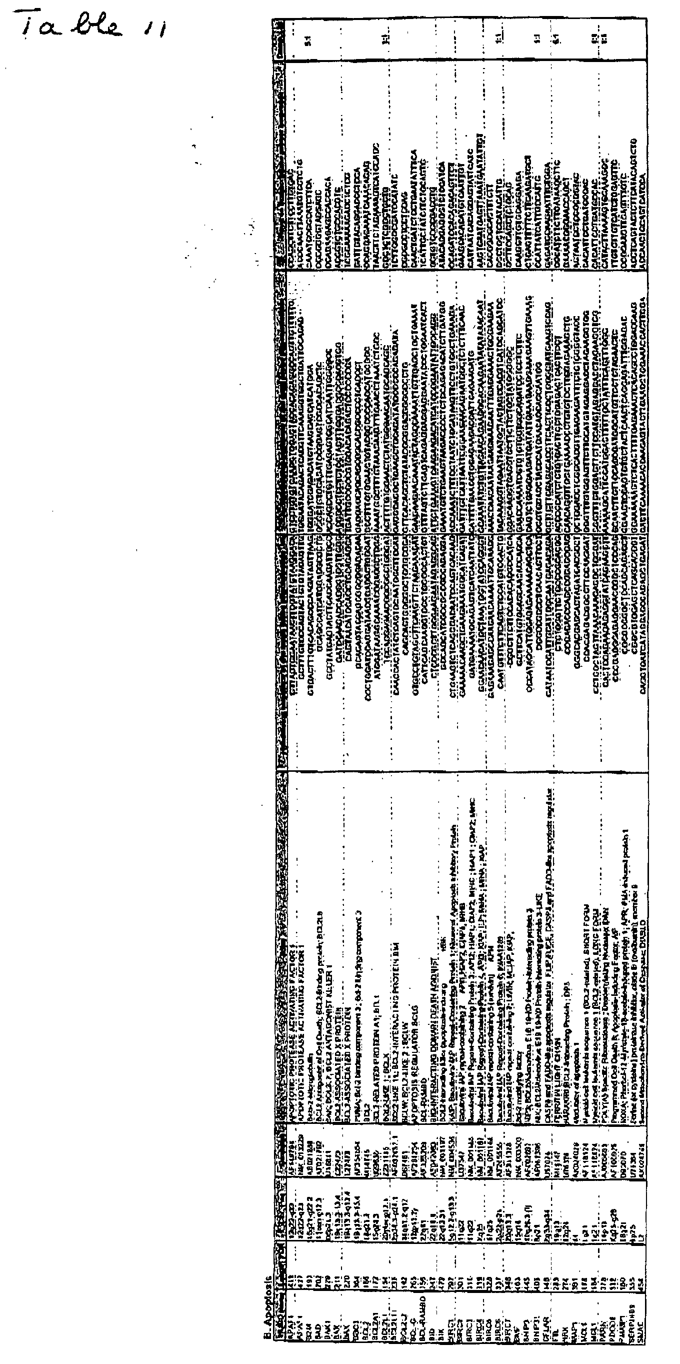

### Brief description of the drawings

Figure 1: Principle of RT-MLPA
   Total RNA is converted to single strand cDNA with gene-specific primers (Target 1, 2..etc). Hemiprobes contain stuffers of variable length. Only upon ligation can the annealed products be amplified via PCR with primers F* and R. This generates labeled DNA fragments of distinct sizes, which are then quantified by capillary sequencer.
Figure 2: General overview of the MLPA technology. After the formation of cDNA, both the MLPA probes hybridize to immediately adjacent target sequences. Following ligation of the probes, the so-derived single strand probe fragment is PCR amplified using primers X and Y. Each probe generates an amplification product of unique length that is detected by electrophoresis. The size of this PCR fragment, dependent on the length of the stuffer region, identifies the amplified gene (details about MLPA can be found on www.mrc-holland.com).
Figure 3: Chemokine mRNA production in human volunteers during low-grade endotoxemia. Eight human volunteers were either given placebo (top panel) or IC14 (bottom panel; 1 mg/kg) i.v. from -2 to -1 hour relative to endotoxin (4 ng/kg) injection.
Figure 4: Cytokine mRNA production in human volunteers during low-grade endotoxemia. Eight human volunteers were either given placebo (top panel) or IC14 (bottom panel, 1 mg/kg) i.v. from -2 to -1 hour relative to endotoxin (4 ng/kg) injection.
Figure 5: IκBα mRNA production in human volunteers during low-grade endotoxemia. Eight human volunteers were either given placebo (top panel) or IC14 (bottom panel, 1 mg/kg) i.v. from -2 to -1 hour relative to endotoxin (4 ng/kg) injection.
Figure 6: Signal ratios in relation to cycle number and fluctuations in expression of one gene. MLPA was performed with the apoptosis probe set on RNA samples from prostate cells. Aliquots were taken after 28, 30, 32, 34 and 40 cycles as indicated, and analyzed as described in Methods. The specific identity of the target genes is irrelevant for these experiments and is therefore not indicated for clarity.
   A. Expression of target genes relative to total signal is indicated for low (<2%, left panel) and high signals (>2%, right panel). As can be seen, the relative ratios between signals are not influenced by the number of PCR cycles.
   B. The cumulative signal plotted against PCR cycle number shows that the total signal strength from cycle 28-34 increases approximately 10-fold, but then remains quite constant.
   C. MLPA was performed for 32 cycles on prostate RNA. Varying concentrations of non-amplifiable competitor oligo targeting BNIP were added as indicated. The decreasing relative response of the BNIP signal is plotted together with responses of all other genes, which can be seen to be constant.
Figure 7: Variations in input RNA and independent duplicates.
   A. MLPA data are independent of input RNA concentration over a wide range of input RNA. Undiluted, 10- and 100-times diluted RNA samples from unfractionated PBLs were analysed by MLPA using the inflammation probe set. The relative expression of an undiluted sample is plotted against the relative expression of the diluted ones. Shown as diamonds (.) is the 1:1 diluted sample, squares (□) depict the 1:10 diluted RNA sample, and triangles (.) represent results of the 1:100 diluted RNA sample.
   B. MLPA was performed independently on duplicate samples from Ramos and Jurkat cells, using the apoptosis probe set. Plotted is the relative expression in 3 independent pairs of duplicate samples. Both probe sets yield highly concordant data for both high and low signals.
Figure 8: Compatibility with other profiling techniques. Comparison of gene expression profiles using RT-MLPA or cDNA-microarrays and standard RTPCR.
   A. Analysis of healthy volunteers during low grade endotoxemia. Fold induction for two representative genes present both in the MLPA probe set as on a custom made micro-array in our institute (manuscript in preparation) is depicted.
   B. Ramos Burkitt lymphoma cells were stimulated with a mAb against the B cell antigen receptor (5 µg/ml) and RNA was isolated at the indicated time points. Results for A1/Bf obtained from RTMLPA are plotted together with RT-PCR results performed on the same samples. Data for housekeeping genes ß2M (MLPA) and GAPDH (RT-PCR) are also represented.
Figure 9: Ligation of distinct Toll-like receptors on PBLs differentially induces inflammatory and apoptosis genes
   Unfractionated PBLs from healthy volunteers were stimulated with the indicated TLR ligands for 2 hrs. RNA samples were subjected to RT-MLPA using the inflammation probe set (A, n=3) and the apoptosis probe set (B, n=2). Results were calculated with reference to housekeeping B2M signal, and then recalculated relative to the medium sample which was set at 1. Selected data from the most relevant changes in expression are shown. Values for control non-CpG treated cells (not shown) were equivalent to those from untreated cells.
Figure 10: Induction of apoptosis in U2OS cells by cytostatic regimens. Osteosarcoma cells (U2OS) were stimulated with actinomycin D, etoposide or fludarabine for 24 hrs. RT-MLPA was performed with the apoptosis probe set, and results are expressed relative to untreated cells (set as 1). Only subsets of data of BH3-only proteins and miscellaneous apoptosis regulators containing the most relevant changes in expression in Puma and Apaf are depicted. The signals of the remaining probes displayed less than twofold changes upon the various stimuli. Results are representative of three experiments.
Figure 11: Suppression of Puma expression prevents drug-induced apoptosis. U2OS cells were transiently transfected with vectors encoding eGFP and empty pSuper RNAi vectors, or together with two RNAi constructs targeting the Puma transcript. Left panel depicts untreated eGPP-expressing cells, middle panels shows cells treated with fludarabine, and the right panel shows the combination of fludarabine and Puma RNAi. Compared to right and left panel, the middle panel show typical signs of apoptosis; cells are rounded off and detached from the well (and therefore sometimes out of focus). Data from one experiment of two is shown.
Figure 12: Base line profiles of individuals Expression profiles for the inflammation probe set in PBLs of 16 different healthy individuals. Gene expression data are depicted as Mean +/- SEM. The fact that 18 out of the 27 inflammatory genes yield a baseline response at resting conditions indicates that not only gene induction but also inhibition of gene expression can be analyzed.
Figure 13: Profile of apoptosis regulators in normal and malignant lymphocytes

Relative expression data from normal naive CD8 T cells and B cells are plotted together with malignant T cells (Jurkat) and B-CLL samples. Results are expressed relative to the total signal in the sample ± SD. Data within a specific cell type are homogeneous, but among distinct cell types clear differences in expression patterns can be observed. Both naive B cells and Jurkat T cells display relatively high levels of Bid transcript, consistent with their sensitivity to CD95-mediated apoptosis(53). Also, the naive tonsil B cell fraction expressed A1/Bfl1, a Bcl2-like protective protein upregulated via CD40-NF.B signaling (54; 55). Lastly, the expression of IAP family member survivin was restricted to dividing cells (also additional data: not shown), consistent with recent insights that it is probably more directly involved in cell cycle regulation rather than in preventing apoptosis (56).

### References

1. Brandtzaeg P, Kierulf P, Gaustad P, Skulberg A, Bruun JN, Halvorsen S, Sorensen E. Plasma endotoxin as a predictor of multiple organ failure and death in systemic meningococcal disease. J Infect Dis. 159:195-204, 1989.
2. Danner RL, Elin RJ, Hosseini JM, Wesley RA, Reilly JM, Parillo JE. Endotoxemia in human septic shock. Chest. 99:169-175, 1991.
3. Ziegler EJ, McCutchan JA, Fierer J, Glauser MP, Sadoff JC, Douglas H, Braude AI. Treatment of gram-negative bacteremia and shock with human antiserum to a mutant Escherichia coli. N Engl J Med. 307:1225-1230, 1982.
4. Wenzel RP, Pinsky MR, Ulevitch RJ, Young L. Current understanding of sepsis. Clin Infect Dis. 22:407-412, 1996.
5. Schumann RR, Leong SR, Flaggs GW, Gray PW, Wright SD, Mathison JC, Tobias PS, Ulevitch RJ. Structure and function of lipopolysaccharide binding protein. Science 249:1429-1431, 1990.
6. Pugin J, Ulevitch RJ, Tobias PS. A critical role for monocytes and CD14 in endotoxin-induced endothelial cell activation. J Exp Med. 178:2193-2200, 1993.
7. Pugin J, Ulevitch RJ, Tobias PS. Activation of endothelial cells by endotoxin: direct versus indirect pathways and the role of CD14. Prog Clin Biol Res. 392:369-373, 1995.
8. Loppnow H, Stelter F, Schonbeck U, Schluter C, Ernst M, Schutt C, Flad HD. Endotoxin activates human vascular smooth muscle cells despite lack of expression of CD14 mRNA or endogenous membrane CD14. Infect Immun. 63:1020-1026, 1995.
9. Qureshi ST, Lariviere L, Leveque G, Clermont S, Moore KJ, Gros P, Malo D. Endotoxin-tolerant mice have mutations in Toll-like receptor 4 (Tlr4). J Exp Med. 189:615-625, 1999.
10. Poltorak A, He X, Smirnova I, Liu MY, Huffel CV, Du X, Birdwell D, Alejos E, Silva M, Galanos C, Freudenberg M, Ricciardi-Castagnoli P, Layton B, Beutler B. Defective LPS signaling in C3H/HeJ and C57BL/10ScCr mice: mutations in Tlr4 gene. Science. 282:2085-2088, 1998.
11. Guha M, Mackman N. LPS induction of gene expression in human monocytes. Cell Signal. 13:85-94, 2001.
12. Ziegler-Heitbrock HW, Ulevitch RJ. CD14: cell surface receptor and differentiation marker. Immunol Today. 14:121-125, 1993.
13. Verbon A, Dekkers PE, ten Hove T, Hack CE, Pribble JP, Turner T, Souza S, Axtelle T, Hoek FJ, van Deventer SJ, van der Poll T. IC14, an anti-CD 14 antibody, inhibits endotoxin-mediated symptoms and inflammatory responses in humans. J Immunol. 166:3599-3605, 2001.
14. Wright SD. CD14 and innate recognition of bacteria. J Immunol. 155:6-8, 1995.
15. Axtelle T, Pribble J. IC14, a CD14 specific monoclonal antibody, is a potential treatment for patients with severe sepsis. J Endotoxin Res. 7:310-314, 2001.
16. Pugin J, Schurer-Maly CC, Leturcq D, Moriarty A, Ulevitch RJ, Tobias PS. Lipopolysaccharide activation of human endothelial and epithelial cells is mediated by lipopolysaccharide-binding protein and soluble CD14. Proc Natl Acad Sci U S A. 90:2744-2748, 1993.
17. Leturcq DJ, Moriarty AM, Talbott G, Winn RK, Martin TR, Ulevitch RJ. Antibodies against CD14 protect primates from endotoxin-induced shock. J Clin Invest. 98:1533-1538, 1996.
18. Olszyna DP, Verbon A, Pribble JP, Turner T, Axtelle T, van Deventer SJH, van der Poll T. IC14, an anti-CD14 antibody, inhibits rises in plasma and cell-associated chemokines in human endotoxemia. Cytokines and chemokines in systemic and urinary tract infection by Escherichia coli. Wageningen: Ponsen & Looijen, 2001.
19. Schouten JP, McElgunn CJ, Waaijer R, Zwijnenburg D, Diepvens F, Pals G. Relative quantification of 40 nucleic acid sequences by Multiplex. Ligation-dependent Probe Amplification. Nucleic Acids Res. 30: e57, 2002.
20. Boom R, Sol CJ, Salimans MM, Jansen CL, Wertheim-van Dillen PM, van der Noordaa J. Rapid and simple method for purification of nucleic acids. J Clin Microbiol. 28:495-503, 1990.
21. Suzuki T, Hashimoto S, Toyoda N, Nagai S, Yamazaki N, Dong HY, Sakai J, Yamashita T, Nukiwa T, Matsushima K. Comprehensive gene expression profile of LPS-stimulated human monocytes by SAGE. Blood 96:2584-2591, 2000.
22. Bazil V, Strominger JL. Shedding as a mechanism of down-modulation of CD14 on stimulated human monocytes. J Immunol. 147:1567-1574, 1991.
23. van der Poll T, Coyle SM, Barbosa K, Braxton CC, Lowry SF. Epinephrine inhibits tumor necrosis factor-alpha and potentiates interleukin 10 production during human endotoxemia. J Clin Invest. 97:713-719, 1996.
24. Suffredini AF, Reda D, Banks SM, Tropea M, Agosti JM, Miller R. Effects of recombinant dimeric TNF receptor on human inflammatory responses following intravenous endotoxin administration. J Immunol. 155:5038-5045, 1995.
25. Franco RF, de Jonge E, Dekkers PE, Timmerman JJ, Spek CA, van Deventer SJ, van Deursen P, van Kerkhoff L, van Gemen B, ten Cate H, van der Poll T, Reitsma PH. The in vivo kinetics of tissue factor messenger RNA expression during human endotoxemia: relationship with activation of coagulation. Blood 96:554-559, 2000.
26. Doggen CJ, Berckmans RJ, Sturk A, et al. C-reactive protein, cardiovascular risk factors and the association with myocardial infarction in men. J Intern Med. 2000;248:406-14.
27. Spek CA, Verbon A, Aberson H, et al. Treatment with an anti-CD 14 monoclonal antibody delays and inhibits lipopolysaccharide-induced gene expression in humans in vivo. J Clin Immunol. 2003;23:132-140.
28. Woolf B. On estimating the relation between blood group and disease. Annals of Human Genetics. 1955;19:251-253.
29. Hamann, D., Baars, P. A., Rep, M. H., Hooibrink, B., Kerkhof-Garde, S. R., Klein, M. R., & van Lier, R. A. (1997) J.Exp.Med. 186, 1407-1418.
30. Lens, S. M., de Jong, R., Hooibrink, B., Koopman, G., Pals, S. T., van Oers, M. H., & van Lier, R. A. (1996) Eur.J.Immunol. 26, 2964-2971.
31. Brummelkamp, T. R., Bernards, R., & Agami, R. (2002) Science 296, 550-553.
32. Takeda, K., Kaisho, T., & Akira, S. (2003) Annu.Rev.Immunol. 21, 335-376.
33. Hornung, V., Rothenfusser, S., Britsch, S., Krug, A., Jahrsdorfer, B., Giese, T., Endres, S., & Hartmann, G. (2002) J Immunol 168, 4531-7.
34. Muzio, M., Bosisio, D., Polentarutti, N., D'Amico, G., Stoppacciaro, A., Mancinelli, R., van't Veer, C., Penton-Rol, G., Ruco, L. P. et al (2000) J Immunol 164, 5998-6004.
35. Sabroe, I., Jones, E. C., Usher, L. R., Whyte, M. K., & Dower, S. K (2002) J Immunol 168, 4701-10.
36. Toshchakov, V., Jones, B. W., Perera, P. Y., Thomas, K., Cody, M. J., Zhang, S., Williams, B. R., Major, J., Hamilton, T. A. et al (2002) Nat Immunol 3, 392-8.
37. Lepe-Zuniga, J. L., Rotbein, J., & Gutterman, J. U. (1989) J Interferon Res 9, 445-56.
38. Visintin, A., Mazzoni, A., Spitzer, J. H., Wyllie, D. H., Dower, S. K., & Segal, D. M. (2001) J Immunol 166, 249-55.
39. Zarember, K. A. & Godowski, P. J. (2002) J Immunol 168, 554-61.
40. Oshiumi, H., Matsumoto, M., Funami, K., Akazawa, T., & Seya, T. (2003) Nat Immunol 4, 161-7.
41. Kannan-Thulasiraman, P. & Shapiro, D. J. (2002) J.Biol.Chem. 277, 41230-41239.
42. Nakano, K. & Vousden, K. H. (2001) Mol.Cell 7, 683-694. 17. Allan, L. A. & Fried, M. (1999) Oncogene 18, 5403-5412.
43. Yu, J., Zhang, L., Hwang, P. M., Kinzler, K. W., & Vogelstein, B. (2001) Mol.Cell 7, 673-682.
44. Li, P., Nijhawan, D., Budihardjo, I., Srinivasula, S. M., Ahmad, M., Alnemri, E. S., & Wang, X. (1997) Cell 91, 479-489.
46. Robles, A. I., Bemmels, N. A., Foraker, A. B., & Harris, C. C. (2001) Cancer Res. 61, 6660-6664.
47. Schuler, M. & Green, D. R. (2001) Biochem.Soc.Trans. 29, 684-688.
48. Yu, J., Wang, Z., Kinzler, K. W., Vogelstein, B., & Zhang, L. (2003) Proc.Natl.Acad.Sci.U.S.A 100, 1931-1936.
49. Brummelkamp, T. R., Bernards, R., & Agami, R. (2002) Science 296, 550-553.
50. Rosenwald, A., Alizadeh, A. A., Widhopf, G., Simon, R., Davis, R. E., Yu, X., Yang, L., Pickeral, O. K., Rassenti, L. Z. et al (2001) J.Exp.Med. 194, 1639-1647.
51. Brachat, A., Pierrat, B., Brungger, A., & Heim, J. (2000) Oncogene 19, 5073-5082.
52. Zhao, H., Hastie, T., Whitfield, M. L., Borresen-Dale, A. L., & Jeffrey, S. S. (2002) BMC.Genomics 3, 31.
53. Krammer, P. H. CD95's deadly mission in the immune system. Nature 407, 789-795 (2000).
54. Lee, H. H., Dadgostar, H., Cheng, Q., Shu, J., & Cheng, G. NF-kappaB-mediated up-regulation of Bcl-x and Bfl-1/A1 is required for CD40 survival signaling in B lymphocytes. Proc.Natl.Acad.Sci.U.S.A 96, 9136-9141 (1999).
55. Grumont, R. J., Rourke, I. J., & Gerondakis, S. Rel-dependent induction of A1 transcription is required to protect B cells from antigen receptor ligation-induced apoptosis. Genes Dev. 13, 400-411 (1999).
56. Reed, J. C. The Survivin saga goes in vivo. J.Clin.Invest 108, 965-969 (2001).

## Claims

1. A method for determining whether a subject is at risk of undergoing an infarction related to atherosclerosis comprising measuring an inflammation parameter in a sample obtained from said subject and determining whether the value of said inflammation parameter is indicative for said risk.

2. A method according to claim 1, wherein said inflammation parameter comprises an inflammation parameter of a cell or derivative thereof present in said sample.

3. A method according to claim 2, wherein said cell comprises a leukocyte.

4. A method according to any one of claims 1-3, wherein said inflammation parameter is measured from RNA present in said sample.

5. A method according to any one of claims 1-4, wherein said inflammation parameter comprises a relative amount of RNA of at least one gene involved in inflammation.

6. A method according to claim 5, wherein said RNA comprises mRNA.

7. A method according to any one of claims 1-6, wherein said inflammation parameter comprises a relative amount of expression products of at least two genes involved in inflammation.

8. A method according to any one of claims 1-7, wherein said inflammation parameter comprises a relative amount of at least one nucleic acid comprising at least a functional part of at least two genes involved in inflammation.

9. A method according to any one of claims 1-8, wherein said inflammation parameter comprises a relative amount of at least one expression product encoded by the gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A.

10. A method according to any one of claims 1-9, wherein said inflammation parameter comprises a relative amount of a nucleic acid comprising at least a functional part of the gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A.

11. A method according to claim 9 or 10, wherein said gene is MIF, PI9, CCL3, PTPN1, BMI1, PDE4B, GSTP1, NFKB1, IL12A, PDGFB, MYC, IL1B, IL8, NFKBIA and/or IL15(1).

12. A method according to any one of claims 9-11, wherein said gene is MIF, PI9, CCL3, PTPN1, and/or BMI1.

13. A method according to any one of claims 1-12, wherein said inflammation parameter is compared with a threshold value.

14. A method according to any one of claims 1-13, wherein said infarction is related to atherosclerosis comprises myocardial infarction.

15. A method according to any one of claims 1-14, wherein said sample is a circulating body fluid.

16. A method according to any one of claims 1-15, wherein said sample is a blood sample.

17. A method according to any one of claims 1-16, wherein said sample is a whole blood sample.

18. Use of an expression product of gene MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A for determining whether a subject is at risk of undergoing an infarction related to atherosclerosis.

19. A method for reducing the risk for a subject of undergoing an infarction related to atherosclerosis comprising at least in part eliminating a controllable risk factor of said subject, **characterized in that** said subject was identified as being at risk for said attack through a method according to any one of claims 1-17.

20. A method according to claim 19, wherein said controllable risk factor comprises lowering cholesterol levels, lowering blood pressure, quitting-smoking, and/or losing weight.

21. A method according to claim 20, wherein said cholesterol levels are lowered by providing said subject with a statin.

22. Use of a statin for the preparation of a medicament for the treatment of a subject at risk of undergoing an infarction related to atherosclerosis, **characterised in that** said subject was identified as being at risk through a method according to any one of claims 1-17.

23. Use of a compound capable of at least in part inhibiting an adverse effect upon atherosclerosis of an expression product encoded by the genes MIF, PI9, CCL3, PTPN1, BMI1, PDE4B, GSTP1, NFKB1, IL12A, PDGFB, MYC, IL1B, ILB, NFKBIA, IL15(1), LTA, IFNG, IL1RN, TNFRSFIA, IL18, CCL4 and/or IL1A for the preparation of a medicament for the treatment of a subject at risk of undergoing an infarction related to atherosclerosis.

24. A method for quantifying at least one target nucleic acid in a sample, comprising
- incubating said sample with at least one probe capable of annealing to said target nucleic acid;
- amplifying annealed probes; and
- quantifying said probes,
**characterized in that** said sample comprises a whole blood sample.

25. A method for quantifying at least one target nucleic acid in a sample, comprising
- incubating said sample with at least one set of at least two oligonucleotides, said at least two oligonucleotides of one set being capable of annealing to (different parts of) a target nucleic acid;
- ligating oligonucleotides of one set that are bound to a target sequence;
- amplifying ligated oligonucleotides; and
- quantifying said oligonucleotides;
**characterized in that** said sample comprises a whole blood sample.

26. A method according to claim 24 or 25, wherein said nucleic acid is RNA.

27. A method for determining whether an expression product is indicative for a disease, comprising determining whether a relative amount of nucleic acid encoding said expression product in a sample of a healthy subject is significantly different from a relative amount of nucleic acid encoding said expression product in a sample of a diseased subject, **characterized in that** said relative amounts are measured by:
- incubating said sample with at least one probe capable of annealing to said nucleic acid encoding said expression product;
- amplifying annealed probes; and
- quantifying said probes.

28. A method according to claim 27, **characterised in that** a multiplex ligation-dependent probe amplification is performed upon a whole blood sample.

29. A method according to claim 27 or 28, wherein said nucleic acid comprises RNA.

30. Use of a compound capable of at least in part inhibiting an adverse effect upon a disease of an expression product identified with a method according to any one of claims 27-29 for the preparation of a medicament for the treatment of a subject suffering from, or at risk of suffering from, said disease.

31. A kit of parts comprising suitable means for performing a method according to any one of claims 1-17 or 24-29.

32. A kit of parts comprising:
- at least one set of MLPA probes capable of annealing to a nucleic acid encoding MIF, PI9, CCL3, PTPN1, BMI1, PDE4B, GSTP1, NFKB1, IL12A, PDGFB, MYC, IL1B, IL8, NFKBIA, IL15(1), LTA, IFNG, IL1RN, TNFRSFIA, IL18, CCL4 and/or IL1A;
- suitable means for performing a MLPA reaction.

33. A kit of parts according to claim 31 or 32, wherein said kit comprises suitable means for isolation of nucleic acids from whole blood.


SEQUENCE LISTING

<110>  Academisch Ziekenhuis bij de Universiteit van Amsterdam
       Leids Universitair Medisch Centrum

<120>  Means and methods for detecting a risk of infarction related to
atherosclerosis

<130>  P63578EP00

<140>  EP 03077299.0
<141>  2003-07-21

<160>  231

<170>  PatentIn version 3.1

<210>  1
<211>  19
<212>  DNA
<213>  Artificial Sequence

<220>
<223>  Primer pr1

<400>  1
gggttcccta agggttgga                                                    19


<210>  2
<211>  23
<212>  DNA
<213>  Artificial Sequence

<220>
<223>  Primer pr2

<400>  2
gtgccagcaa gatccaatct aga                                               23


<210>  3
<211>  19
<212>  DNA
<213>  Artificial Sequence

<220>
<223>  Oligonucleotide

<400>  3
gtgaaggtcg gagtcaacg                                                    19


<210>  4
<211>  19
<212>  DNA
<213>  Artificial Sequence

<220>
<223>  Oligonucleotide

<400>  4
tgaagacgcc agtggactc                                                    19


<210>  5
<211>  19
<212>  DNA
<213>  Artificial Sequence

<220>
<223>  Puma sequence

<400>  5
agacaggaat ccacggctt                                                    19


<210>  6
<211>  19
<212>  DNA
<213>  Artificial Sequence

<220>
<223>  Puma sequence

<400>  6
cgagatggag cccaattag                                                    19


<210>  7
<211>  30
<212>  DNA
<213>  Artificial Sequence

<220>
<223>  Hybridizing sequence of the small probe oligonucleotide

<400>  7
gtgactttgt cacagcccaa gatagttaag                                        30


<210>  8
<211>  24
<212>  DNA
<213>  Artificial Sequence

<220>
<223>  Hybridizing sequence of the small probe oligonucleotide

<400>  8
tttatgcagc tcatccttct gctg                                              24


<210>  9

<211>  21
<212>  DNA
<213>  Artificial Sequence

<220>
<223>  Hybridizing sequence of the small probe oligonucleotide

<400>  9
cggcagacca gcatgacaga t                                                21


<210>  10
<211>  26
<212>  DNA
<213>  Artificial Sequence

<220>
<223>  Hybridizing sequence of the small probe oligonucleotide

<400>  10
cagagttcac accttacctg gagaca                                           26


<210>  11
<211>  24
<212>  DNA
<213>  Artificial Sequence

<220>
<223>  Hybridizing sequence of the small probe oligonucleotide

<400>  11
gggctcactc aaagcctcct gcct                                             24


<210>  12
<211>  30
<212>  DNA
<213>  Artificial Sequence

<220>
<223>  Hybridizing sequence of the small probe oligonucleotide

<400>  12
gatgacttcg aaaagctgac taattattcg                                       30


<210>  13
<211>  21
<212>  DNA
<213>  Artificial Sequence

<220>
<223>  Hybridizing sequence of the small probe oligonucleotide

<400>  13
gctgaggcta cggcgctgtc a                                                21

<210> 14
<211> 26
<212> DNA
<213> Artificial Sequence

<220>
<223> Hybridizing sequence of the small probe oligonucleotide

<400> 14
catgctggca gttattgatg agctga 26

<210> 15
<211> 24
<212> DNA
<213> Artificial Sequence

<220>
<223> Hybridizing sequence of the small probe oligonucleotide

<400> 15
cattcagtgt caaaagcagc agag 24

<210> 16
<211> 24
<212> DNA
<213> Artificial Sequence

<220>
<223> Hybridizing sequence of the small probe oligonucleotide

<400> 16
gctggtcaac atcacccaga acca 24

<210> 17
<211> 27
<212> DNA
<213> Artificial Sequence

<220>
<223> Hybridizing sequence of the small probe oligonucleotide

<400> 17
ggcattcatg tcttcatttt gggctgt 27

<210> 18
<211> 21
<212> DNA
<213> Artificial Sequence

<220>
<223> Hybridizing sequence of the small probe oligonucleotide

<400>  18
accatagatt tgtgcagctg t                                                21

<210>  19
<211>  33
<212>  DNA
<213>  Artificial Sequence

<220>
<223>  Hybridizing sequence of the small probe oligonucleotide

<400>  19
agatatgact gattctgact gtagagataa tgc                                   33

<210>  20
<211>  23
<212>  DNA
<213>  Artificial Sequence

<220>
<223>  Hybridizing sequence of the small probe oligonucleotide

<400>  20
agaccaacca gtgctgctga agg                                              23

<210>  21
<211>  27
<212>  DNA
<213>  Artificial Sequence

<220>
<223>  Hybridizing sequence of the small probe oligonucleotide

<400>  21
agctgatggc cctaaacaga tgaagtg                                          27

<210>  22
<211>  23
<212>  DNA
<213>  Artificial Sequence

<220>
<223>  Hybridizing sequence of the small probe oligonucleotide

<400>  22
tccagcaaga tgcaagcctt cag                                              23

<210>  23
<211>  24
<212>  DNA
<213>  Artificial Sequence

<220>
<223>  Hybridizing sequence of the small probe oligonucleotide

<400>  23
gttttacatg cccaagaagg ccac                                              24


<210>  24
<211>  21
<212>  DNA
<213>  Artificial Sequence

<220>
<223>  Hybridizing sequence of the small probe oligonucleotide

<400>  24
tgaacagcct cacagagcag a                                                 21


<210>  25
<211>  27
<212>  DNA
<213>  Artificial Sequence

<220>
<223>  Hybridizing sequence of the small probe oligonucleotide

<400>  25
ccgtaacaga catctttgct gcctcca                                           27


<210>  26
<211>  24
<212>  DNA
<213>  Artificial Sequence

<220>
<223>  Hybridizing sequence of the small probe oligonucleotide

<400>  26
tgcttccaat ctggattcaa tgag                                              24


<210>  27
<211>  24
<212>  DNA
<213>  Artificial Sequence

<220>
<223>  Hybridizing sequence of the small probe oligonucleotide

<400>  27
gccttcctga tttctgcagc tctg                                              24


<210>  28

<211>  27
<212>  DNA
<213>  Artificial Sequence

<220>
<223>  Hybridizing sequence of the small probe oligonucleotide

<400>  28
caccctcaaa cctgctgctc acctcat                                          27


<210>  29
<211>  21
<212>  DNA
<213>  Artificial Sequence

<220>
<223>  Hybridizing sequence of the small probe oligonucleotide

<400>  29
gcctgcgcat cagcccggac a                                                21


<210>  30
<211>  21
<212>  DNA
<213>  Artificial Sequence

<220>
<223>  Hybridizing sequence of the small probe oligonucleotide

<400>  30
caccaccagc agcgactctg a                                                21


<210>  31
<211>  17
<212>  DNA
<213>  Artificial Sequence

<220>
<223>  Hybridizing sequence of the small probe oligonucleotide

<400>  31
ggggaccggc agctggg                                                     17


<210>  32
<211>  23
<212>  DNA
<213>  Artificial Sequence

<220>
<223>  Hybridizing sequence of the small probe oligonucleotide

<400>  32
agggccgaaa gacctatccc act                                              23

<210>  33
<211>  28
<212>  DNA
<213>  Artificial Sequence

<220>
<223>  Hybridizing sequence of the small probe oligonucleotide

<400>  33
cactccagaa gagaggtatc agaagctt                                          28


<210>  34
<211>  24
<212>  DNA
<213>  Artificial Sequence

<220>
<223>  Hybridizing sequence of the small probe oligonucleotide

<400>  34
tgaagatcac ctggccaagg agct                                              24


<210>  35
<211>  24
<212>  DNA
<213>  Artificial Sequence

<220>
<223>  Hybridizing sequence of the small probe oligonucleotide

<400>  35
ctcgtggaag aaggagcctg ggtt                                              24


<210>  36
<211>  23
<212>  DNA
<213>  Artificial Sequence

<220>
<223>  Hybridizing sequence of the small probe oligonucleotide

<400>  36
gttgcagtgc attgtgttgc agg                                               23


<210>  37
<211>  24
<212>  DNA
<213>  Artificial Sequence

<220>
<223>  Hybridizing sequence of the small probe oligonucleotide

<400>  37
cttaaatgcc gcaccctacg gcat                                              24


<210>  38
<211>  21
<212>  DNA
<213>  Artificial Sequence

<220>
<223>  Hybridizing sequence of the small probe oligonucleotide

<400>  38
aagggctcgc tcagccagat g                                                 21


<210>  39
<211>  26
<212>  DNA
<213>  Artificial Sequence

<220>
<223>  Hybridizing sequence of the small probe oligonucleotide

<400>  39
ggctctctgc aaccagttct ctgcat                                            26


<210>  40
<211>  23
<212>  DNA
<213>  Artificial Sequence

<220>
<223>  Hybridizing sequence of the small probe oligonucleotide

<400>  40
gcgctctcag caccaatggg ctc                                               23


<210>  41
<211>  24
<212>  DNA
<213>  Artificial Sequence

<220>
<223>  Hybridizing sequence of the small probe oligonucleotide

<400>  41
ctcagggact tgctcagcca gatt                                              24


<210>  42
<211>  21
<212>  DNA
<213>  Artificial Sequence

<220>
<223>  Hybridizing sequence of the small probe oligonucleotide

<400>  42
cggcgtggag ctcagcacgg t                                                21


<210>  43
<211>  28
<212>  DNA
<213>  Artificial Sequence

<220>
<223>  Hybridizing sequence of the small probe oligonucleotide

<400>  43
tagctgcact gagtgtcact gtcagaac                                         28


<210>  44
<211>  24
<212>  DNA
<213>  Artificial Sequence

<220>
<223>  Hybridizing sequence of the small probe oligonucleotide

<400>  44
gtgacaagcc tgtagcccat gttg                                             24


<210>  45
<211>  24
<212>  DNA
<213>  Artificial Sequence

<220>
<223>  Hybridizing sequence of the small probe oligonucleotide

<400>  45
atgttaaggg cactgaggac tcag                                             24


<210>  46
<211>  31
<212>  DNA
<213>  Artificial Sequence

<220>
<223>  Hybridizing sequence of the long probe oligonucleotide

<400>  46
tgggatcgag acatgtaagc agcatcatgg a                                     31


<210>  47

<211>  34
<212>  DNA
<213>  Artificial Sequence

<220>
<223>  Hybridizing sequence of the long probe oligonucleotide

<400>  47
atgctgccaa tggctctaat gaagatagag gaga                                34


<210>  48
<211>  37
<212>  DNA
<213>  Artificial Sequence

<220>
<223>  Hybridizing sequence of the long probe oligonucleotide

<400>  48
ttctaccact ccaaacgccg gctgatcttc tccaaga                             37


<210>  49
<211>  35
<212>  DNA
<213>  Artificial Sequence

<220>
<223>  Hybridizing sequence of the long probe oligonucleotide

<400>  49
aacctcggac agccaacaat tcagagtttt gaaca                               35


<210>  50
<211>  37
<212>  DNA
<213>  Artificial Sequence

<220>
<223>  Hybridizing sequence of the long probe oligonucleotide

<400>  50
atacgggcag ctccccaagt tccaggacgg agacctc                             37


<210>  51
<211>  34
<212>  DNA
<213>  Artificial Sequence

<220>
<223>  Hybridizing sequence of the long probe oligonucleotide

<400>  51
gtaactgact tgaatgtcca acgcaaagca atac                                34

<210> 52
<211> 34
<212> DNA
<213> Artificial Sequence

<220>
<223> Hybridizing sequence of the long probe oligonucleotide

<400> 52
tcgatttctt ccctgtgaaa acaagagcaa ggcc 34

<210> 53
<211> 35
<212> DNA
<213> Artificial Sequence

<220>
<223> Hybridizing sequence of the long probe oligonucleotide

<400> 53
tgcaggccct gaatttcaac agtgagactg tgcca 35

<210> 54
<211> 31
<212> DNA
<213> Artificial Sequence

<220>
<223> Hybridizing sequence of the long probe oligonucleotide

<400> 54
gctcttctga cccccaaggg gtgacgtgcg g 31

<210> 55
<211> 40
<212> DNA
<213> Artificial Sequence

<220>
<223> Hybridizing sequence of the long probe oligonucleotide

<400> 55
gaaggctccg ctctgcaatg gcagcatggt atggagcatc 40

<210> 56
<211> 37
<212> DNA
<213> Artificial Sequence

<220>
<223> Hybridizing sequence of the long probe oligonucleotide

<400>  56
ttcagtgcag ggcttcctaa aacagaagcc aactggg                                37

<210>  57
<211>  43
<212>  DNA
<213>  Artificial Sequence

<220>
<223>  Hybridizing sequence of the long probe oligonucleotide

<400>  57
accccggacc atatttatta taagtatgta taaagatagc cag                         43

<210>  58
<211>  38
<212>  DNA
<213>  Artificial Sequence

<220>
<223>  Hybridizing sequence of the long probe oligonucleotide

<400>  58
agatgcctga gatacccaaa accatcacag gtagtgag                               38

<210>  59
<211>  34
<212>  DNA
<213>  Artificial Sequence

<220>
<223>  Hybridizing sequence of the long probe oligonucleotide

<400>  59
ctccttccag gacctggacc tctgccctct ggat                                   34

<210>  60
<211>  38
<212>  DNA
<213>  Artificial Sequence

<220>
<223>  Hybridizing sequence of the long probe oligonucleotide

<400>  60
aatctgggat gttaaccaga agaccttcta tctgagga                               38

<210>  61
<211>  43
<212>  DNA
<213>  Artificial Sequence

<220>
<223>  Hybridizing sequence of the long probe oligonucleotide

<400>  61
agaactgaaa catcttcagt gtctagaaga agaactcaaa cct                43


<210>  62
<211>  37
<212>  DNA
<213>  Artificial Sequence

<220>
<223>  Hybridizing sequence of the long probe oligonucleotide

<400>  62
agaacacaac tgagaaggaa accttctgca gggctgc                37


<210>  63
<211>  43
<212>  DNA
<213>  Artificial Sequence

<220>
<223>  Hybridizing sequence of the long probe oligonucleotide

<400>  63
gagacttgcc tggtgaaaat catcactggt cttttggagt ttg                43


<210>  64
<211>  37
<212>  DNA
<213>  Artificial Sequence

<220>
<223>  Hybridizing sequence of the long probe oligonucleotide

<400>  64
tgtgaaggtg cagttttgcc aaggagtgct aaagaac                37


<210>  65
<211>  37
<212>  DNA
<213>  Artificial Sequence

<220>
<223>  Hybridizing sequence of the long probe oligonucleotide

<400>  65
tggagacccc agcaagcaga actcactgct ctggaga                37


<210>  66

<211>  37
<212>  DNA
<213>  Artificial Sequence

<220>
<223>  Hybridizing sequence of the long probe oligonucleotide

<400>  66
gggtctacat caactattac gacatgaacg cggccaa                                37


<210>  67
<211>  34
<212>  DNA
<213>  Artificial Sequence

<220>
<223>  Hybridizing sequence of the long probe oligonucleotide

<400>  67
ggaggaacaa gaagatgagg aagaaatcga tgtt                                   34


<210>  68
<211>  29
<212>  DNA
<213>  Artificial Sequence

<220>
<223>  Hybridizing sequence of the long probe oligonucleotide

<400>  68
agatcgggaa aaagagctaa tccgccaag                                         29


<210>  69
<211>  35
<212>  DNA
<213>  Artificial Sequence

<220>
<223>  Hybridizing sequence of the long probe oligonucleotide

<400>  69
gtcaagatct gtaactacga gggaccagcc aagat                                  35


<210>  70
<211>  39
<212>  DNA
<213>  Artificial Sequence

<220>
<223>  Hybridizing sequence of the long probe oligonucleotide

<400>  70
aaaaagcatt ccatggactt tttgctattt cagtttggc                              39

<210> 71
<211> 37
<212> DNA
<213> Artificial Sequence

<220>
<223> Hybridizing sequence of the long probe oligonucleotide

<400> 71
ggaagacctg aacaaatggg gtcttaacat ctttaat 37

<210> 72
<211> 31
<212> DNA
<213> Artificial Sequence

<220>
<223> Hybridizing sequence of the long probe oligonucleotide

<400> 72
ccctgaccat tgctgagccg gccatgatcg c 31

<210> 73
<211> 35
<212> DNA
<213> Artificial Sequence

<220>
<223> Hybridizing sequence of the long probe oligonucleotide

<400> 73
attgggaagg gcacctgtgc tggttgcact tgctt 35

<210> 74
<211> 37
<212> DNA
<213> Artificial Sequence

<220>
<223> Hybridizing sequence of the long probe oligonucleotide

<400> 74
cgaaagcatg agtcaagaca ctgaagttag aagtcgg 37

<210> 75
<211> 37
<212> DNA
<213> Artificial Sequence

<220>
<223> Hybridizing sequence of the long probe oligonucleotide

<400>  75
caatcaatgc cccagtcacc tgctgttata acttcac                                37

<210>  76
<211>  32
<212>  DNA
<213>  Artificial Sequence

<220>
<223>  Hybridizing sequence of the long probe oligonucleotide

<400>  76
cacttgctgc tgacacgccg accgcctgct gc                                     32

<210>  77
<211>  32
<212>  DNA
<213>  Artificial Sequence

<220>
<223>  Hybridizing sequence of the long probe oligonucleotide

<400>  77
agaccctccc accgcctgct gcttttctta ca                                     32

<210>  78
<211>  34
<212>  DNA
<213>  Artificial Sequence

<220>
<223>  Hybridizing sequence of the long probe oligonucleotide

<400>  78
cagtttccat tccaatcacc tgctgcttta acgt                                   34

<210>  79
<211>  43
<212>  DNA
<213>  Artificial Sequence

<220>
<223>  Hybridizing sequence of the long probe oligonucleotide

<400>  79
ggaaaaaagt ctcacttttg agaaactcac agcctggacc aag                         43

<210>  80
<211>  36
<212>  DNA
<213>  Artificial Sequence

<220>
<223>  Hybridizing sequence of the long probe oligonucleotide

<400>  80
tcagttacca tctgcaaaaa ggtgtcctgc cccatc                                36


<210>  81
<211>  37
<212>  DNA
<213>  Artificial Sequence

<220>
<223>  Hybridizing sequence of the long probe oligonucleotide

<400>  81
tagcaaaccc tcaagctgag gggcagctcc agtggct                               37


<210>  82
<211>  37
<212>  DNA
<213>  Artificial Sequence

<220>
<223>  Hybridizing sequence of the long probe oligonucleotide

<400>  82
gcaccacagt gctgttgccc ctggtcattt tctttgg                               37


<210>  83
<211>  17
<212>  DNA
<213>  Artificial Sequence

<220>
<223>  reverse transcriptase primer sequence

<400>  83
caaatgcggc atcttca                                                     17


<210>  84
<211>  21
<212>  DNA
<213>  Artificial Sequence

<220>
<223>  reverse transcriptase primer sequence

<400>  84
ctcttatctt catctgcaac c                                                21


<210>  85

<211>  18
<212>  DNA
<213>  Artificial Sequence

<220>
<223>  reverse transcriptase primer sequence

<400>  85
ggattagggc ttcctctt                                                    18


<210>  86
<211>  20
<212>  DNA
<213>  Artificial Sequence

<220>
<223>  reverse transcriptase primer sequence

<400>  86
acttttgttc ccacctgttc                                                  20


<210>  87
<211>  18
<212>  DNA
<213>  Artificial Sequence

<220>
<223>  reverse transcriptase primer sequence

<400>  87
ggactggtac agggtgag                                                    18


<210>  88
<211>  17
<212>  DNA
<213>  Artificial Sequence

<220>
<223>  reverse transcriptase primer sequence

<400>  88
gacagttcag ccatcac                                                     17


<210>  89
<211>  17
<212>  DNA
<213>  Artificial Sequence

<220>
<223>  reverse transcriptase primer sequence

<400>  89
aggcattctt cacctgc                                                     17

<210> 90
<211> 18
<212> DNA
<213> Artificial Sequence

<220>
<223> reverse transcriptase primer sequence

<400> 90
cggttcttca agggagga 18


<210> 91
<211> 20
<212> DNA
<213> Artificial Sequence

<220>
<223> reverse transcriptase primer sequence

<400> 91
gcagagagtg tagcagctcc 20


<210> 92
<211> 15
<212> DNA
<213> Artificial Sequence

<220>
<223> reverse transcriptase primer sequence

<400> 92
gggctgcaca gtaca 15


<210> 93
<211> 23
<212> DNA
<213> Artificial Sequence

<220>
<223> reverse transcriptase primer sequence

<400> 93
aatcacttat tacattcacc cag 23


<210> 94
<211> 21
<212> DNA
<213> Artificial Sequence

<220>
<223> reverse transcriptase primer sequence

<400>  94
gttacagcca tacctctagg c                                                21

<210>  95
<211>  20
<212>  DNA
<213>  Artificial Sequence

<220>
<223>  reverse transcriptase primer sequence

<400>  95
cagaagaaga ggaggttggt                                                  20

<210>  96
<211>  19
<212>  DNA
<213>  Artificial Sequence

<220>
<223>  reverse transcriptase primer sequence

<400>  96
ggagattcgt agctggatg                                                   19

<210>  97
<211>  19
<212>  DNA
<213>  Artificial Sequence

<220>
<223>  reverse transcriptase primer sequence

<400>  97
ccagcaacta gttggttgt                                                   19

<210>  98
<211>  19
<212>  DNA
<213>  Artificial Sequence

<220>
<223>  reverse transcriptase primer sequence

<400>  98
ttagcacttc ctccagagg                                                   19

<210>  99
<211>  16
<212>  DNA
<213>  Artificial Sequence

<220>
<223>  reverse transcriptase primer sequence

<400>  99
actgccggag cacagt                                                    16


<210>  100
<211>  24
<212>  DNA
<213>  Artificial Sequence

<220>
<223>  reverse transcriptase primer sequence

<400>  100
gtactctagg tatacctcaa actc                                           24


<210>  101
<211>  25
<212>  DNA
<213>  Artificial Sequence

<220>
<223>  reverse transcriptase primer sequence

<400>  101
gtatgtcttt atgcactgac atcta                                          25


<210>  102
<211>  16
<212>  DNA
<213>  Artificial Sequence

<220>
<223>  reverse transcriptase primer sequence

<400>  102
acggtccgtg tttgct                                                    16


<210>  103
<211>  17
<212>  DNA
<213>  Artificial Sequence

<220>
<223>  reverse transcriptase primer sequence

<400>  103
agttgttcca gcccaca                                                   17


<210>  104

<211>  19
<212>  DNA
<213>  Artificial Sequence

<220>
<223>  reverse transcriptase primer sequence

<400>  104
gcctcttttc cacagaaac                                                    19


<210>  105
<211>  17
<212>  DNA
<213>  Artificial Sequence

<220>
<223>  reverse transcriptase primer sequence

<400>  105
gtctgctgca gagctgc                                                      17


<210>  106
<211>  17
<212>  DNA
<213>  Artificial Sequence

<220>
<223>  reverse transcriptase primer sequence

<400>  106
ccaggtccac ctcgatc                                                      17


<210>  107
<211>  21
<212>  DNA
<213>  Artificial Sequence

<220>
<223>  reverse transcriptase primer sequence

<400>  107
catacttaaa agtgcaaagg c                                                 21


<210>  108
<211>  20
<212>  DNA
<213>  Artificial Sequence

<220>
<223>  reverse transcriptase primer sequence

<400>  108
ttgtgagaat atccagccac                                                   20

<210>  109
<211>  15
<212>  DNA
<213>  Artificial Sequence

<220>
<223>  reverse transcriptase primer sequence

<400>  109
gcgcgtcttg cactc                                                        15


<210>  110
<211>  19
<212>  DNA
<213>  Artificial Sequence

<220>
<223>  reverse transcriptase primer sequence

<400>  110
ttccacattc aatcaaagc                                                    19


<210>  111
<211>  16
<212>  DNA
<213>  Artificial Sequence

<220>
<223>  reverse transcriptase primer sequence

<400>  111
agacttcccc ccacga                                                       16


<210>  112
<211>  21
<212>  DNA
<213>  Artificial Sequence

<220>
<223>  reverse transcriptase primer sequence

<400>  112
tgcactgaga tcttcctatt g                                                 21


<210>  113
<211>  18
<212>  DNA
<213>  Artificial Sequence

<220>
<223>  reverse transcriptase primer sequence

<400>  113
cgggaggtgt agctgaag                                                    18


<210>  114
<211>  16
<212>  DNA
<213>  Artificial Sequence

<220>
<223>  reverse transcriptase primer sequence

<400>  114
cttcctcgcg gtgtaa                                                      16


<210>  115
<211>  23
<212>  DNA
<213>  Artificial Sequence

<220>
<223>  reverse transcriptase primer sequence

<400>  115
tctggatagg aattttccta ttg                                              23


<210>  116
<211>  25
<212>  DNA
<213>  Artificial Sequence

<220>
<223>  reverse transcriptase primer sequence

<400>  116
acctcagtac tcttcataca gtctg                                            25


<210>  117
<211>  16
<212>  DNA
<213>  Artificial Sequence

<220>
<223>  reverse transcriptase primer sequence

<400>  117
cattggagca gggcat                                                      16


<210>  118
<211>  13
<212>  DNA
<213>  Artificial Sequence

<220>
<223>  reverse transcriptase primer sequence

<400>  118
cccggcggtt cag                                                          13


<210>  119
<211>  19
<212>  DNA
<213>  Artificial Sequence

<220>
<223>  reverse transcriptase primer sequence

<400>  119
gggataaaag gcaaagacc                                                    19


<210>  120
<211>  30
<212>  DNA
<213>  Artificial Sequence

<220>
<223>  Hybridizing sequence of the small probe oligonucleotide

<400>  120
gttagtggaa taacttcgta tgtaaggaca                                        30


<210>  121
<211>  27
<212>  DNA
<213>  Artificial Sequence

<220>
<223>  Hybridizing sequence of the small probe oligonucleotide

<400>  121
gctttgtccc agtactgtgt agagttg                                           27


<210>  122
<211>  30
<212>  DNA
<213>  Artificial Sequence

<220>
<223>  Hybridizing sequence of the small probe oligonucleotide

<400>  122
gtgactttgt cacagcccaa gatagttaag                                        30


<210>  123

<211>  21
<212>  DNA
<213>  Artificial Sequence

<220>
<223>  Hybridizing sequence of the small probe oligonucleotide

<400>  123
gcagccatca tggaggcgct g                                                21


<210>  124
<211>  27
<212>  DNA
<213>  Artificial Sequence

<220>
<223>  Hybridizing sequence of the small probe oligonucleotide

<400>  124
gcctatgagt acttcaccaa gattgcc                                          27


<210>  125
<211>  23
<212>  DNA
<213>  Artificial Sequence

<220>
<223>  Hybridizing sequence of the small probe oligonucleotide

<400>  125
gatccaagac cagggtggtt ggg                                              23


<210>  126
<211>  23
<212>  DNA
<213>  Artificial Sequence

<220>
<223>  Hybridizing sequence of the small probe oligonucleotide

<400>  126
cagtaacatg gagctgcaga gga                                              23


<210>  127
<211>  24
<212>  DNA
<213>  Artificial Sequence

<220>
<223>  Hybridizing sequence of the small probe oligonucleotide

<400>  127
gcacagtacg agcggcggag acaa                                             24

<210> 128
<211> 30
<212> DNA
<213> Artificial Sequence

<220>
<223> Hybridizing sequence of the small probe oligonucleotide

<400> 128
ccctggatcc aggataacgg aggctgggat 30

<210> 129
<211> 26
<212> DNA
<213> Artificial Sequence

<220>
<223> Hybridizing sequence of the small probe oligonucleotide

<400> 129
atggataagg caaaacggag gctggg 26

<210> 130
<211> 23
<212> DNA
<213> Artificial Sequence

<220>
<223> Hybridizing sequence of the small probe oligonucleotide

<400> 130
tccaggagaa cggcggctgg gat 23

<210> 131
<211> 30
<212> DNA
<213> Artificial Sequence

<220>
<223> Hybridizing sequence of the small probe oligonucleotide

<400> 131
caaccactat ctcagtgcaa tggcttccat 30

<210> 132
<211> 21
<212> DNA
<213> Artificial Sequence

<220>
<223> Hybridizing sequence of the small probe oligonucleotide

<400>  132
cagcagtggg ggctgggcgg a                                                21

<210>  133
<211>  30
<212>  DNA
<213>  Artificial Sequence

<220>
<223>  Hybridizing sequence of the small probe oligonucleotide

<400>  133
gtgcctgtag cttcaagttc taagaaagat                                       30

<210>  134
<211>  27
<212>  DNA
<213>  Artificial Sequence

<220>
<223>  Hybridizing sequence of the small probe oligonucleotide

<400>  134
cattcagcaa ggtggctggg gcactgt                                          27

<210>  135
<211>  26
<212>  DNA
<213>  Artificial Sequence

<220>
<223>  Hybridizing sequence of the small probe oligonucleotide

<400>  135
ctcccgcttg ggaagaatag aggcag                                           26

<210>  136
<211>  24
<212>  DNA
<213>  Artificial Sequence

<220>
<223>  Hybridizing sequence of the small probe oligonucleotide

<400>  136
gccagcatcg ccgccgccag agga                                             24

<210>  137
<211>  30
<212>  DNA
<213>  Artificial Sequence

<220>
<223>  Hybridizing sequence of the small probe oligonucleotide

<400>  137
ctgaagtctc agggacaatc cagtcacaag                                        30


<210>  138
<211>  30
<212>  DNA
<213>  Artificial Sequence

<220>
<223>  Hybridizing sequence of the small probe oligonucleotide

<400>  138
gaaaaacaag ctgaagaaat ggcatcagat                                        30


<210>  139
<211>  27
<212>  DNA
<213>  Artificial Sequence

<220>
<223>  Hybridizing sequence of the small probe oligonucleotide

<400>  139
gatgaaaatg cagagtcatc aattatc                                           27


<210>  140
<211>  29
<212>  DNA
<213>  Artificial Sequence

<220>
<223>  Hybridizing sequence of the small probe oligonucleotide

<400>  140
ggaacaacat gctaaatggt atccagggt                                         29


<210>  141
<211>  30
<212>  DNA
<213>  Artificial Sequence

<220>
<223>  Hybridizing sequence of the small probe oligonucleotide

<400>  141
gagaaagagc caagaacaaa attgcaaagg                                        30


<210>  142

<211>  29
<212>  DNA
<213>  Artificial Sequence

<220>
<223>  Hybridizing sequence of the small probe oligonucleotide

<400>  142
caatgtttct tcagtctcca tgtccactg                                          29


<210>  143
<211>  24
<212>  DNA
<213>  Artificial Sequence

<220>
<223>  Hybridizing sequence of the small probe oligonucleotide

<400>  143
cggcttcttc cacacaggcc atca                                               24


<210>  144
<211>  24
<212>  DNA
<213>  Artificial Sequence

<220>
<223>  Hybridizing sequence of the small probe oligonucleotide

<400>  144
gcttcatgtg cagcaacacc agca                                               24


<210>  145
<211>  29
<212>  DNA
<213>  Artificial Sequence

<220>
<223>  Hybridizing sequence of the small probe oligonucleotide

<400>  145
cccatagcat tggagagaaa aacagctca                                          29


<210>  146
<211>  21
<212>  DNA
<213>  Artificial Sequence

<220>
<223>  Hybridizing sequence of the small probe oligonucleotide

<400>  146
cggccggcct caacagttcc t                                                  21

<210>  147
<211>  30
<212>  DNA
<213>  Artificial Sequence

<220>
<223>  Hybridizing sequence of the small probe oligonucleotide

<400>  147
gataatcgat tgcattggca atgagacaga                                        30


<210>  148
<211>  21
<212>  DNA
<213>  Artificial Sequence

<220>
<223>  Hybridizing sequence of the small probe oligonucleotide

<400>  148
ctctgggttc tgcccgcacg g                                                 21


<210>  149
<211>  21
<212>  DNA
<213>  Artificial Sequence

<220>
<223>  Hybridizing sequence of the small probe oligonucleotide

<400>  149
ccgagaccca gccggagcga g                                                 21


<210>  150
<211>  24
<212>  DNA
<213>  Artificial Sequence

<220>
<223>  Hybridizing sequence of the small probe oligonucleotide

<400>  150
gggcacgagc actagatcac ggct                                              24


<210>  151
<211>  23
<212>  DNA
<213>  Artificial Sequence

<220>
<223>  Hybridizing sequence of the small probe oligonucleotide

<400> 151
ccacgagacg gccttccaag gat 23

<210> 152
<211> 29
<212> DNA
<213> Artificial Sequence

<220>
<223> Hybridizing sequence of the small probe oligonucleotide

<400> 152
cctggctagt taaacaaaga ggctgggat 29

<210> 153
<211> 28
<212> DNA
<213> Artificial Sequence

<220>
<223> Hybridizing sequence of the small probe oligonucleotide

<400> 153
cactccagaa gagaggtatc agaagctt 28

<210> 154
<211> 26
<212> DNA
<213> Artificial Sequence

<220>
<223> Hybridizing sequence of the small probe oligonucleotide

<400> 154
cccgaggcag aggaaccggc tcccag 26

<210> 155
<211> 21
<212> DNA
<213> Artificial Sequence

<220>
<223> Hybridizing sequence of the small probe oligonucleotide

<400> 155
cgcgcgggct ccagcagagc t 21

<210> 156
<211> 21
<212> DNA
<213> Artificial Sequence

<220>
<223>  Hybridizing sequence of the small probe oligonucleotide

<400>  156
cggcgtggag ctcagcacgg t                                                21


<210>  157
<211>  29
<212>  DNA
<213>  Artificial Sequence

<220>
<223>  Hybridizing sequence of the small probe oligonucleotide

<400>  157
caggtgatca taggagccag agctgagat                                        29


<210>  158
<211>  43
<212>  DNA
<213>  Artificial Sequence

<220>
<223>  Hybridizing sequence of the long probe oligonucleotide

<400>  158
gtcctgtgtg aaggtggagt accacagagg ccagttgttt ttg                        43


<210>  159
<211>  40
<212>  DNA
<213>  Artificial Sequence

<220>
<223>  Hybridizing sequence of the long probe oligonucleotide

<400>  159
tggaatacag actcacgttc aaaggtggct gattgcagag                            40


<210>  160
<211>  31
<212>  DNA
<213>  Artificial Sequence

<220>
<223>  Hybridizing sequence of the long probe oligonucleotide

<400>  160
tgggatcgag acatgtaagc agcatcatgg a                                     31


<210>  161

<211>  31
<212>  DNA
<213>  Artificial Sequence

<220>
<223>  Hybridizing sequence of the long probe oligonucleotide

<400>  161
gggctgtgga gatccggagt cgccacagct c                                    31


<210>  162
<211>  34
<212>  DNA
<213>  Artificial Sequence

<220>
<223>  Hybridizing sequence of the long probe oligonucleotide

<400>  162
accagcctgt ttgagagtgg catcaattgg ggcc                                 34


<210>  163
<211>  35
<212>  DNA
<213>  Artificial Sequence

<220>
<223>  Hybridizing sequence of the long probe oligonucleotide

<400>  163
acggcctcct ctcctacttt gggacgccca cgtgg                                35


<210>  164
<211>  32
<212>  DNA
<213>  Artificial Sequence

<220>
<223>  Hybridizing sequence of the long probe oligonucleotide

<400>  164
tgattgccgc cgtggacaca gactcccccc ga                                   32


<210>  165
<211>  31
<212>  DNA
<213>  Artificial Sequence

<220>
<223>  Hybridizing sequence of the long probe oligonucleotide

<400>  165
gaggagcagc agcggcaccg cccctcaccc t                                    31

<210>  166
<211>  34
<212>  DNA
<213>  Artificial Sequence

<220>
<223>  Hybridizing sequence of the long probe oligonucleotide

<400>  166
gcctttgtgg aactgtacgg ccccagcatg cggc                                    34


<210>  167
<211>  38
<212>  DNA
<213>  Artificial Sequence

<220>
<223>  Hybridizing sequence of the long probe oligonucleotide

<400>  167
aaaatggctt tgtaaagaag tttgaaccta aatctggc                                38


<210>  168
<211>  35
<212>  DNA
<213>  Artificial Sequence

<220>
<223>  Hybridizing sequence of the long probe oligonucleotide

<400>  168
acttttgtgg aactctatgg gaacaatgca gcagc                                   35


<210>  169
<211>  37
<212>  DNA
<213>  Artificial Sequence

<220>
<223>  Hybridizing sequence of the long probe oligonucleotide

<400>  169
gaggcaggct gaacctgcag atatgcgccc agagata                                 37


<210>  170
<211>  31
<212>  DNA
<213>  Artificial Sequence

<220>
<223>  Hybridizing sequence of the long probe oligonucleotide

<400>  170
gttcacagct ctatacgggg acggggccct g                                    31


<210>  171
<211>  43
<212>  DNA
<213>  Artificial Sequence

<220>
<223>  Hybridizing sequence of the long probe oligonucleotide

<400>  171
gaagaagaac aaatactagc caaaattgtt gagctgctga aat                       43


<210>  172
<211>  43
<212>  DNA
<213>  Artificial Sequence

<220>
<223>  Hybridizing sequence of the long probe oligonucleotide

<400>  172
gtttagtctt gagtcagagg aggaggaata ccctggaatc act                       43


<210>  173
<211>  41
<212>  DNA
<213>  Artificial Sequence

<220>
<223>  Hybridizing sequence of the long probe oligonucleotide

<400>  173
attctgaaag tcaagaagac atcatccgga atattgccag g                         41


<210>  174
<211>  43
<212>  DNA
<213>  Artificial Sequence

<220>
<223>  Hybridizing sequence of the long probe oligonucleotide

<400>  174
gaaatgtctg aagtaagacc cctctccaga gacatcttga tgg                       43


<210>  175
<211>  43
<212>  DNA
<213>  Artificial Sequence

<220>
<223>  Hybridizing sequence of the long probe oligonucleotide

<400>  175
accaaatctt tcctaatctg gataagttcc tgtgcctgaa aga                43


<210>  176
<211>  43
<212>  DNA
<213>  Artificial Sequence

<220>
<223>  Hybridizing sequence of the long probe oligonucleotide

<400>  176
gatttgtcat taattcggaa gaacagaatg gctctctttc aac                43


<210>  177
<211>  34
<212>  DNA
<213>  Artificial Sequence

<220>
<223>  Hybridizing sequence of the long probe oligonucleotide

<400>  177
cattttgaac ctggagaaga ccattcagaa gatg                          34


<210>  178
<211>  44
<212>  DNA
<213>  Artificial Sequence

<220>
<223>  Hybridizing sequence of the long probe oligonucleotide

<400>  178
gcaaatatct gttagaacag aagggacaag aatatataaa caat               44


<210>  179
<211>  43
<212>  DNA
<213>  Artificial Sequence

<220>
<223>  Hybridizing sequence of the long probe oligonucleotide

<400>  179
aaaccaacaa taagaagaaa gaatttgagg aaactgcgaa gaa                43


<210>  180

<211>  44
<212>  DNA
<213>  Artificial Sequence

<220>
<223>  Hybridizing sequence of the long probe oligonucleotide

<400>  180
tacaaaggta gaattaatgc tactagccac gtcatccagc atcc                        44


<210>  181
<211>  34
<212>  DNA
<213>  Artificial Sequence

<220>
<223>  Hybridizing sequence of the long probe oligonucleotide

<400>  181
ggacaaggtg aggtgcttct tctgctatgg gggc                                   34


<210>  182
<211>  37
<212>  DNA
<213>  Artificial Sequence

<220>
<223>  Hybridizing sequence of the long probe oligonucleotide

<400>  182
gaaccaaaat cgtgtgtggt ggcagatcct cctcttc                                37


<210>  183
<211>  44
<212>  DNA
<213>  Artificial Sequence

<220>
<223>  Hybridizing sequence of the long probe oligonucleotide

<400>  183
cagtctgagg aagatgatat tgaaagaagg aaagaagttg aaag                        44


<210>  184
<211>  31
<212>  DNA
<213>  Artificial Sequence

<220>
<223>  Hybridizing sequence of the long probe oligonucleotide

<400>  184
gggtggagct acccatgaac agcagcaatg g                                      31

<210>  185
<211>  43
<212>  DNA
<213>  Artificial Sequence

<220>
<223>  Hybridizing sequence of the long probe oligonucleotide

<400>  185
gcttcttcga gacaccttca cttccctggg ctatgaagtc cag                43


<210>  186
<211>  37
<212>  DNA
<213>  Artificial Sequence

<220>
<223>  Hybridizing sequence of the long probe oligonucleotide

<400>  186
acccccatct ctgtgacttc ctggagactc acttcct                       37


<210>  187
<211>  40
<212>  DNA
<213>  Artificial Sequence

<220>
<223>  Hybridizing sequence of the long probe oligonucleotide

<400>  187
caacaggttg gtgaaaaccc tgtgtccttg gagaaagctg                    40


<210>  188
<211>  40
<212>  DNA
<213>  Artificial Sequence

<220>
<223>  Hybridizing sequence of the long probe oligonucleotide

<400>  188
gctggacctc ggcacgttga caagatttct ctggggtacc                    40


<210>  189
<211>  41
<212>  DNA
<213>  Artificial Sequence

<220>
<223>  Hybridizing sequence of the long probe oligonucleotide

<400> 189
gggtttgtgg agttcttcca tgtagaggac ctagaaggtg g 41

<210> 190
<211> 39
<212> DNA
<213> Artificial Sequence

<220>
<223> Hybridizing sequence of the long probe oligonucleotide

<400> 190
aaaaagcatt ccatggactt tttgctattt cagtttggc 39

<210> 191
<211> 38
<212> DNA
<213> Artificial Sequence

<220>
<223> Hybridizing sequence of the long probe oligonucleotide

<400> 191
gcaacttgtt ccagcgatgg catgttcctc tagaactc 38

<210> 192
<211> 40
<212> DNA
<213> Artificial Sequence

<220>
<223> Hybridizing sequence of the long probe oligonucleotide

<400> 192
ggaagtcgag tgtgctactc aactcaggag atttggagac 40

<210> 193
<211> 43
<212> DNA
<213> Artificial Sequence

<220>
<223> Hybridizing sequence of the long probe oligonucleotide

<400> 193
ggaaaaaagt ctcacttttg agaaactcac agcctggacc aag 43

<210> 194
<211> 44
<212> DNA
<213> Artificial Sequence

<220>
<223>  Hybridizing sequence of the long probe oligonucleotide

<400>  194
gacttcaaaa caccaagagt acttgaagct ggaaaccact tgga                        44


<210>  195
<211>  19
<212>  DNA
<213>  Artificial Sequence

<220>
<223>  reverse transcriptase primer sequence

<400>  195
ccagcttctt ccttgtgac                                                    19


<210>  196
<211>  21
<212>  DNA
<213>  Artificial Sequence

<220>
<223>  reverse transcriptase primer sequence

<400>  196
acccaactta aatgtcctct g                                                 21


<210>  197
<211>  17
<212>  DNA
<213>  Artificial Sequence

<220>
<223>  reverse transcriptase primer sequence

<400>  197
caaatgcggc atcttca                                                      17


<210>  198
<211>  14
<212>  DNA
<213>  Artificial Sequence

<220>
<223>  reverse transcriptase primer sequence

<400>  198
cgcggggtag gagc                                                         14


<210>  199

<211>  17
<212>  DNA
<213>  Artificial Sequence

<220>
<223>  reverse transcriptase primer sequence

<400>  199
ccagaagagc caccaca                                                    17


<210>  200
<211>  15
<212>  DNA
<213>  Artificial Sequence

<220>
<223>  reverse transcriptase primer sequence

<400>  200
acggtctgcc acgtg                                                      15


<210>  201
<211>  18
<212>  DNA
<213>  Artificial Sequence

<220>
<223>  reverse transcriptase primer sequence

<400>  201
tcggaaaaag acctctcg                                                   18


<210>  202
<211>  19
<212>  DNA
<213>  Artificial Sequence

<220>
<223>  reverse transcriptase primer sequence

<400>  202
gattgtacag gaccctcca                                                  19


<210>  203
<211>  20
<212>  DNA
<213>  Artificial Sequence

<220>
<223>  reverse transcriptase primer sequence

<400>  203
ccaggagaaa tcaaacagag                                                 20

<210>  204
<211>  24
<212>  DNA
<213>  Artificial Sequence

<220>
<223>  reverse transcriptase primer sequence

<400>  204
taacttctag aaaagtcatc cagc                                              24


<210>  205
<211>  15
<212>  DNA
<213>  Artificial Sequence

<220>
<223>  reverse transcriptase primer sequence

<400>  205
ggctctcggc tgctg                                                        15


<210>  206
<211>  18
<212>  DNA
<213>  Artificial Sequence

<220>
<223>  reverse transcriptase primer sequence

<400>  206
tcttgggcga tccatatc                                                     18


<210>  207
<211>  14
<212>  DNA
<213>  Artificial Sequence

<220>
<223>  reverse transcriptase primer sequence

<400>  207
cgcgcctcct ccag                                                         14


<210>  208
<211>  23
<212>  DNA
<213>  Artificial Sequence

<220>
<223>  reverse transcriptase primer sequence

<400>  208
caactgatct cctgaatatt tca                                              23


<210>  209
<211>  21
<212>  DNA
<213>  Artificial Sequence

<220>
<223>  reverse transcriptase primer sequence

<400>  209
tcattgctat cttctgcagt g                                                21


<210>  210
<211>  15
<212>  DNA
<213>  Artificial Sequence

<220>
<223>  reverse transcriptase primer sequence

<400>  210
gctgtccccg acctg                                                       15


<210>  211
<211>  20
<212>  DNA
<213>  Artificial Sequence

<220>
<223>  reverse transcriptase primer sequence

<400>  211
atacaggagg gtctccatca                                                  20


<210>  212
<211>  20
<212>  DNA
<213>  Artificial Sequence

<220>
<223>  reverse transcriptase primer sequence

<400>  212
ccagatccac agacagttct                                                  20


<210>  213
<211>  20
<212>  DNA
<213>  Artificial Sequence

<220>
<223>  reverse transcriptase primer sequence

<400>  213
gaagcacaca tgtcaattgt                                                    20


<210>  214
<211>  23
<212>  DNA
<213>  Artificial Sequence

<220>
<223>  reverse transcriptase primer sequence

<400>  214
cattaatcac aggagtattc atc                                                23


<210>  215
<211>  26
<212>  DNA
<213>  Artificial Sequence

<220>
<223>  reverse transcriptase primer sequence

<400>  215
aagtgaatga gttaaatgaa tattgt                                             26


<210>  216
<211>  16
<212>  DNA
<213>  Artificial Sequence

<220>
<223>  reverse transcriptase primer sequence

<400>  216
cacggcgcac tttctt                                                        16


<210>  217
<211>  17
<212>  DNA
<213>  Artificial Sequence

<220>
<223>  reverse transcriptase primer sequence

<400>  217
gcctgctcca tacattg                                                       17


<210>  218

<211>  16
<212>  DNA
<213>  Artificial Sequence

<220>
<223>  reverse transcriptase primer sequence

<400>  218
gcttccagct ctgcag                                                        16


<210>  219
<211>  18
<212>  DNA
<213>  Artificial Sequence

<220>
<223>  reverse transcriptase primer sequence

<400>  219
caaggttgtg caggaaga                                                      18


<210>  220
<211>  22
<212>  DNA
<213>  Artificial Sequence

<220>
<223>  reverse transcriptase primer sequence

<400>  220
ctgagttttt cttcaagatg ct                                                 22


<210>  221
<211>  18
<212>  DNA
<213>  Artificial Sequence

<220>
<223>  reverse transcriptase primer sequence

<400>  221
ccattatcat tgccattg                                                      18


<210>  222
<211>  20
<212>  DNA
<213>  Artificial Sequence

<220>
<223>  reverse transcriptase primer sequence

<400>  222
gagatgcaag aatttctgga                                                    20

<210> 223
<211> 21
<212> DNA
<213> Artificial Sequence

<220>
<223> reverse transcriptase primer sequence

<400> 223
cccatcttct tgataagctt c 21


<210> 224
<211> 17
<212> DNA
<213> Artificial Sequence

<220>
<223> reverse transcriptase primer sequence

<400> 224
gaaaacggga accagct 17


<210> 225
<211> 18
<212> DNA
<213> Artificial Sequence

<220>
<223> reverse transcriptase primer sequence

<400> 225
agtaatcctc cgcggtac 18


<210> 226
<211> 17
<212> DNA
<213> Artificial Sequence

<220>
<223> reverse transcriptase primer sequence

<400> 226
cacattcctg atgccac 17


<210> 227
<211> 21
<212> DNA
<213> Artificial Sequence

<220>
<223> reverse transcriptase primer sequence

<400>  227
catacttaaa agtgcaaagg c                                                21

<210>  228
<211>  21
<212>  DNA
<213>  Artificial Sequence

<220>
<223>  reverse transcriptase primer sequence

<400>  228
ttgtcttgtc atctggagtt c                                                21

<210>  229
<211>  18
<212>  DNA
<213>  Artificial Sequence

<220>
<223>  reverse transcriptase primer sequence

<400>  229
ccggaagttc agtttgtc                                                    18

<210>  230
<211>  25
<212>  DNA
<213>  Artificial Sequence

<220>
<223>  reverse transcriptase primer sequence

<400>  230
acctcagtac tcttcataca gtctg                                            25

<210>  231
<211>  18
<212>  DNA
<213>  Artificial Sequence

<220>
<223>  reverse transcriptase primer sequence

<400>  231
accaactgca gtcatcca                                                    18